# EUROPEAN PATENT APPLICATION

(11) **EP 3 978 510 A1**
(43) Date of publication of application: **06.04.2022**
(21) Application number: 20814786.8
(22) Date of filing: 28.05.2020
(51) Int. Cl.: C07K 7/06, C07K 7/64, C12N 15/09, C12Q 1/04

(54) **NOROVIRUS-BINDING PEPTIDE**

(30) Priority: 29.05.2019 JP 2019100756
(71) Applicant: Kao Corporation, Chuo-ku Tokyo 103-8210 (JP)
(72) Inventor: ITO, Yoshitaka, Haga-gun, Tochigi 321-3497 (JP); MORIMOTO, Takuya, Haga-gun, Tochigi 321-3497 (JP); KUMACHI, Shigefumi, Saitama-shi, Saitama 338-8570 (JP); NEMOTO, Naoto, Saitama-shi, Saitama 338-8570 (JP)
(74) Representative: Vossius & Partner Patentanwälte Rechtsanwälte mbB
(86) International application number: PCT/JP2020/021237
(87) International publication number: WO 2020/241798

(57) **Abstract**

A peptide that specifically binds to norovirus, which is useful for detection and infection control of norovirus is provided. A norovirus-binding peptide consisting of an amino acid sequence selected from the group consisting of SEQ ID NOs: 1 to 269.

## Description

### Field of the Invention

The present invention relates to a norovirus-binding peptide having affinity to norovirus.

### Background of the Invention

Norovirus is a virus that has been called SRSV (Small Round Structured Virus) and also called NLV (Norwalk-like virus), and is classified into five categories from GI (genogroup I) to GV (genogroup V) based on the genotypes, among which GI, GII, and GIV infect humans. Norovirus is a virus that propagates in human intestinal cells and causes food poisoning with symptoms such as diarrhea, vomiting, abdominal pain, nausea, and fever. The main source of infection is food, and raw oysters are often a problem. In addition, in recent years, human to human transmission through excrement etc. of a virus carrier is also increasing.

Currently, as the detection of norovirus, in addition to observation with an electron microscope, there are a method using an antibody and a method of measuring the amount of an amplification product of norovirus RNA. Furthermore, recently, a polypeptide consisting of 18 amino acids that has affinity to norovirus and is useful for detection of norovirus has also been found, but it has been reported that the bonding strength is low compared to previously reported norovirus antibodies (Non Patent Literature 1).

However, a method for detecting RNA requires reverse transcription and an amplification step, and the operation is complicated and takes time and cost. Antibodies also have problems: the specificity is low in some cases; and since animals or culture cells are used for producing and manufacturing antibodies, the quality is unstable and the cost is high.

Accordingly, there is a demand for developing a more effective and simpler norovirus-specific detection method and a prophylactic and therapeutic method for norovirus infection.

### [Non Patent Literature 1]

Hye Jin Hwang, et al., Biosensors and Bioelectronics, 2017, 87, 164-170

### Summary of the Invention

The present invention relates to the following 1) to 4) :
1) a norovirus-binding peptide consisting of an amino acid sequence selected from the group consisting of SEQ ID NOs: 1 to 269;
2) a norovirus-binding peptide consisting of an amino acid sequence selected from the group consisting of SEQ ID NOs: 1 to 269 with a cysteine residue added to either or both of an N-terminus and a C-terminus thereof;
3) a method for detecting norovirus comprising using the norovirus-binding peptide of the above 1) or 2); and
4) a norovirus detection kit comprising the norovirus-binding peptide of the above 1) or 2).

### Brief Description of the Drawings

[Figure 1] Figure 1 is a schematic diagram showing a production flow of VLPs.
[Figure 2] Figure 2 is a schematic diagram showing an outline of screening for norovirus-binding peptides.
[Figure 3] Figure 3 is a schematic diagram showing mRNA-linker conjugates (A: for selection, B: for analysis).
[Figure 4] Figure 4 shows results of evaluation of interaction by ELISA.

### Detailed Description of the Invention

The present invention relates to provision of a peptide that specifically binds to norovirus, which is useful for specific detection and infection control of norovirus.

The present inventors constructed a cDNA library containing a 10¹⁴-digit number of cDNAs and succeeded in obtaining peptides that specifically bind to norovirus from the library by a cDNA display method, and accomplished the present invention.

According to the present invention, norovirus-binding peptides having high affinity to norovirus are provided. According to the peptides of the present invention, norovirus can be specifically detected with a high sensitivity, and infection of humans with norovirus can be controlled.

The norovirus-binding peptide of the present invention is a peptide composed of 6 amino acids, consisting of an amino acid sequence selected from the group consisting of SEQ ID NOs: 1 to 269 (Table 16).

The peptides have been screened from the cDNA library containing a 10¹⁴-digit number of cDNAs by a cDNA display method through in vitro selection using norovirus as a target molecule and are norovirus-binding peptide aptamers having an ability of specifically binding to norovirus. The peptides are each composed of 6 amino acids of the library sequences consisting of 34.4% of hydrophobic amino acids and 65.6% of hydrophilic amino acids. The norovirus-binding peptides of the present invention are those that frequently appear (the appearance frequency is six or more in Examples) among norovirus-binding peptides screened by in vitro selection using norovirus as a target molecule. A norovirus-binding peptide consisting of an amino acid sequence selected from the group consisting of SEQ ID NOs: 1 to 101 that more frequently appears (the appearance frequency is eight or more) is preferred. A norovirus-binding peptide consisting of an amino acid sequence selected from the group consisting of SEQ ID NOs: 1 to 52 (the appearance frequency is ten or more) is more preferred, and a norovirus-binding peptide consisting of an amino acid sequence selected from the group consisting of SEQ ID NOs: 1 to 28 (the appearance frequency is 12 or more) is further preferred. A norovirus-binding peptide consisting of an amino acid sequence selected from the group consisting of SEQ ID NOs: 1 to 12, 14 to 17, 19, 21, and 28 is further preferred, and a norovirus-binding peptide consisting of an amino acid sequence selected from the group consisting of SEQ ID NOs: 1, 4, 14, 15, 17, 21, and 28 is further more preferred.

The peptide of the present invention encompasses, as an aspect, a peptide consisting of an amino acid sequence selected from the group consisting of SEQ ID NOs: 1 to 269 with a cysteine residue added to either or both of an N-terminus and a C-terminus thereof. The peptide having cysteine residues at both terminuses can form a cyclic peptide through a disulfide bond of the cysteine residues.

In addition, the peptide of the present invention encompasses, as another aspect, a peptide consisting of an amino acid sequence selected from the group consisting of SEQ ID NOs: 1 to 269, or an amino acid sequence with a cysteine residue added to either or both of the N-terminus and the C-terminus thereof, wherein arbitrary 1 to 20 amino acid residues are further added to either or both of the N-terminus and the C-terminus of the peptide, as long as the ability of specifically binding with norovirus is maintained.

Viruses that belong to norovirus infecting humans are classified into three gene groups of Genogroup I (GI), Genogroup II (GII), and Genogroup IV (GIV) at present, and 90% or more of reported infection cases are in the GII group. It is inferred there is a serotype corresponding to each genotype. In the present invention, the norovirus encompasses viruses belonging to such norovirus. Empty virus-like particles (VLPs) that are extremely similar to virus particles can be produced by incorporating the structural protein region of a norovirus genome into baculovirus and expressing it in insect cells. The VLPs have the structure of norovirus itself and have antigenicity equivalent to that of virus particles, but do not have the genomic RNA therein, being empty and not having infectivity. Accordingly, in the present invention, norovirus encompasses such VLPs.

Examples of the VLPs include VLPs produced using the norovirus genome such as a GII.4 Saga1 strain (Genbank No. AB447456), a GII.4 Sydney strain (Genbank No. JX459908.1), a GII.3 TCH strain (Genbank No. KF006265), a GII.2 Ehime strain (Genbank No. LC145808), a GII.17 Kawasaki strain (Genbank No. AB983218), and a GII.17 Saitama strain (Genbank No. KJ196286.1).

The peptide of the present invention can be produced using norovirus (empty virus-like particles: VLPs) as a target molecule by in vitro evolution method known in the art, for example, by a cDNA display method (Nucleic Acid Research, vol. 37, No. 16, e108 (2009)). That is, the peptide can be produced by constructing a cDNA library containing cDNAs (library of peptide-linker-mRNA/cDNA conjugates) and subjecting it to in vitro selection by a cDNA display method.

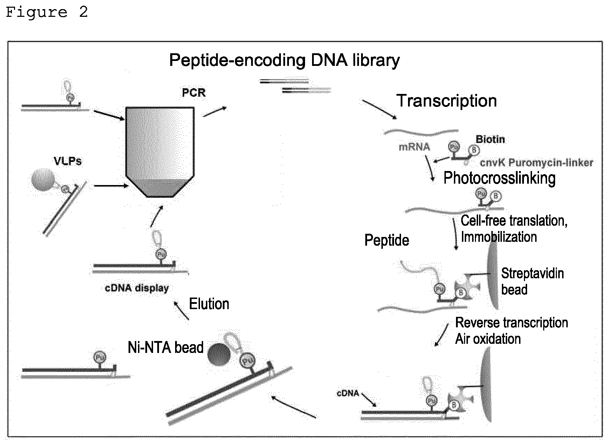

Specifically, the peptide can be produced by the following steps a) to c) (see Figure 2):
a) a step of preparing DNA fragments (construct) encoding a desired random peptide library;
b) a cDNA display-producing step of producing peptide-linker-mRNA/cDNA in vitro with a cDNA display method using the construct prepared in the above step; and
c) a selection step of mixing the cDNA displays obtained in the above step with VLPs, collecting the cDNA displays bound to the VLPs, and screening for VLP-bound cDNA displays.

### a. Step of preparing construct

As a construct for producing a norovirus-binding peptide, DNA fragments including a primer region, a promoter region, an untranslated region, a random region, and a tag region from the 5' end toward the 3' end and encoding a desired random peptide library are constructed. Here, the DNA sequence used as the primer region may be a commercially available general one. As the promoter region, for example, T7 or SP6 can be used. As the untranslated region, for example, an **Ω** region can be used.

For the random region, the DNA is constituted such that hydrophobic amino acids of alanine, valine, leucine, isoleucine, methionine, proline, phenylalanine, and tryptophan is 34.4%, polar amino acids of glycine, serine, threonine, asparagine, glutamine, tyrosine, and cysteine is 31.3%, basic amino acids of lysine, arginine, and histidine is 15.6%, and acidic amino acids of aspartic acid and glutamic acid is 6.3%.

### b. cDNA display-producing step

The production of cDNA display includes, as shown in Figure 2, a mRNA preparation step (b1), a linker-mRNA conjugate formation step (b2), a peptide-linker-mRNA conjugate formation step (b3), a particle-binding step (b4), a cDNA display formation step (b5), a peptide crosslinking step (b6), and a cDNA display release step (b7) .

In the mRNA preparation step (b1), mRNA is prepared from the above-described construct by transcription. Then, in (b2), a linker-mRNA conjugate is formed by binding the mRNA obtained in the mRNA preparation step to a linker to which puromycin is bound.

Subsequently, in (b3), a peptide-linker-mRNA conjugate is formed by binding a peptide having an amino acid sequence corresponding to the mRNA sequence translated by a cell-free translation system to puromycin.

Subsequently, in the particle-binding step (b4), the peptide-linker-mRNA conjugate obtained as in above is bound to magnetic particles.

Subsequently, in (b5), the mRNA of the peptide-linker-mRNA conjugate bound to the magnetic particles is reversely transcribed to form cDNA to obtain peptide-linker-mRNA/cDNA ("cDNA display").

Subsequently, in (b6), cysteines on the N-terminus and the C-terminus of the random region of the peptide in the cDNA display obtained in the above step are crosslinked by a crosslinking reaction.

Subsequently, in the complex release step (b7), the cDNA display obtained in the above step is released from the magnetic particles and is purified as needed.

### c. Selection step of VLP-bound cDNA displays

The selection of VLP-bound cDNA displays includes a solution addition step (c1), a separation step (c2), and a collection step (c3).

In the solution addition step (c1), a cDNA display-containing solution is added to a VLP solution. Continuously, in the separation step (c2), the mixture solution of the VLP and cDNA display solutions is subjected to, for example, centrifugation at 130,000 × g for 5 minutes to precipitate the VLPs. Thus, the cDNA display not bound to the VLPs is separated.
Subsequently, in the collection step (c3), the cDNA display bound to the VLPs is collected together with the VLPs.

The peptide of the present invention can be selected from a predetermined DNA library in vitro as described above.

In addition, the peptide of the present invention can be produced by a known method for manufacturing a peptide, for example, by a chemical synthesis method such as a liquid-phase method, a solid-phase method, or a hybrid method of a liquid-phase method and a solid-phase method; or a genetic recombination method.

Since the peptide of the present invention specifically binds to norovirus, it is possible to verify that norovirus is present or not present in a sample by bringing the peptide into contact with the sample that contains or may contain norovirus.

That is, for example, norovirus in a sample can be detected using the peptide of the present invention instead of an anti-norovirus antibody in an immunoassay such as an ELISA method.

The peptide of the present invention when used as a detection reagent may be labeled to be detectable. In labeling of the peptide, for example, not only enzymes such as peroxidase and alkaline phosphatase, but also radioactive materials, fluorescent materials, luminescent materials, etc. are used. In addition, nanoparticles such as colloidal gold and quantum dots, can also be used. In an immunoassay, the peptide of the present invention can also be detected by labeling the peptide with biotin and binding avidin or streptavidin labeled with an enzyme or the like thereto.

Among the immunoassays, an ELISA method using an enzyme label is preferred in the point that it can simply and rapidly measure an antigen. When norovirus is detected by an ELISA method using the peptide of the present invention, for example, norovirus is immobilized on a solid support, and a peptide previously labeled with biotin is bound thereto. After washing, an avidin-modified enzyme is allowed to bind to the biotin and is then allowed to react with an enzyme substrate to cause color development, and the norovirus can be detected by measuring the absorbance. Alternatively, the peptide of the present invention is solid-phased, and norovirus is bound thereto. After washing, an anti-norovirus antibody labeled with an enzyme or an anti-norovirus antibody and an enzyme-labeled secondary antibody is allowed to bind thereto, and the norovirus can be detected by reacting an enzyme substrate to cause color development and measuring the absorbance.

As the enzyme substrate, when the enzyme is alkaline phosphatase, for example, p-nitrophenyl phosphate (NPP) can be used, and when the enzyme is peroxidase, for example, 3,3',5,5'-tetramethylbenzidine can be used. As the solid support, an insoluble support in a shape of, for example, a bead, microplate, test tube, stick, or test piece made of a material such as polystyrene, polycarbonate, polyvinyl toluene, polypropylene, polyethylene, polyvinyl chloride, nylon, polymethacrylate, latex, gelatin, agarose, cellulose, Sepharose, glass, metal, ceramic, or a magnetic material, can be used. Immobilization of the peptide of the present invention and so on to the solid support can be performed by binding through a known method such as a physical adsorption method, a chemical bond method, or a method of simultaneously performing these methods.

The peptide of the present invention can be a component of a norovirus detection kit. The detection kit can include, in addition to the peptide of the present invention, a reagent and an instrument necessary for detection such as an antibody, a solid support, a buffer solution, an enzyme reaction stopping solution, and a microplate reader.

The sample that is an object of the detection kit is not particularly limited as long as, for example, the sample contains or may contain norovirus, and examples thereof include clinical materials such as feces and vomit collected from a patient, a separated virus culture solution, food such as oyster, and tap and sewage water.

The peptide of the present invention can specifically bind to, for example, the capsid protein of norovirus to inhibit the binding of the virus to a cell. Accordingly, the peptide of the present invention can be used as an anti-norovirus formulation or a medicine for preventing or treating norovirus.

When the peptide of the present invention is used as a medicine, it may be an oral form or a parenteral form and can be appropriately used in combination with known pharmaceutically acceptable avirulent carrier and diluent. Although typical examples of the parenteral administration include an injection, the peptide can also be administered by inhalation with a spray agent, etc.

Regarding the above-described embodiments, the present invention discloses the following aspects:
<1> a norovirus-binding peptide consisting of an amino acid sequence selected from the group consisting of SEQ ID NOs: 1 to 269;
<2> a norovirus-binding peptide consisting of an amino acid sequence selected from the group consisting of SEQ ID NOs: 1 to 269 with a cysteine residue bound to either or both of the N-terminus and the C-terminus thereof;
<3> the norovirus-binding peptide of <2>, wherein the cysteine residue is bound to the N-terminus and the C-terminus of the amino acid sequence selected from the group consisting of SEQ ID NOs: 1 to 269;
<4> the norovirus-binding peptide of <3>, wherein the cysteine residue bound to the N-terminus and the cysteine residue bound to the C-terminus of the peptide consisting of an amino acid sequence selected from the group consisting of SEQ ID NOs: 1 to 269 are linked to each other via a disulfide bond to form a ring;
<5> a norovirus-binding peptide consisting of an amino acid sequence of the peptide according to any one of <1> to <4> with 1 to 20 amino acids bound to either or both of the N-terminus and the C-terminus of the peptide;
<6> a method for detecting norovirus comprising using the norovirus-binding peptide according to any one of <1> to <5>;
<7> a norovirus detection kit comprising the norovirus-binding peptide according to any one of <1> to <5>;
<8> the norovirus-binding peptide according to any one of <1> to <6> or the norovirus detection kit according to <7>, wherein the amino acid sequence selected from the group consisting of SEQ ID NOs: 1 to 269 is an amino acid sequence selected from the group consisting of SEQ ID NOs: 1 to 101;
<9> the norovirus-binding peptide according to any one of <1> to <6> or the norovirus detection kit according to <7>, wherein the amino acid sequence selected from the group consisting of SEQ ID NOs: 1 to 269 is an amino acid sequence selected from the group consisting of SEQ ID NOs: 1 to 52;
<10> the norovirus-binding peptide according to any one of <1> to <6> or the norovirus detection kit according to <7>, wherein the amino acid sequence selected from the group consisting of SEQ ID NOs: 1 to 269 is an amino acid sequence selected from the group consisting of SEQ ID NOs: 1 to 28;
<11> the norovirus-binding peptide according to any one of <1> to <6> or the norovirus detection kit according to <7>, wherein the amino acid sequence selected from the group consisting of SEQ ID NOs: 1 to 269 is an amino acid sequence selected from the group consisting of SEQ ID NOs: 1 to 12, 14 to 17, 19, 21, and 28; and
<12> the norovirus-binding peptide according to any one of <1> to <6> or the norovirus detection kit according to <7>, wherein the amino acid sequence selected from the group consisting of SEQ ID NOs: 1 to 269 is an amino acid sequence selected from the group consisting of SEQ ID NOs: 1, 4, 14, 15, 17, 21, and 28.

### Examples

The present invention will now be more specifically described by examples.

### Reference Example: Production of VLPs

### (1) Introduction of VP1 and VP2 genes into pDEST8

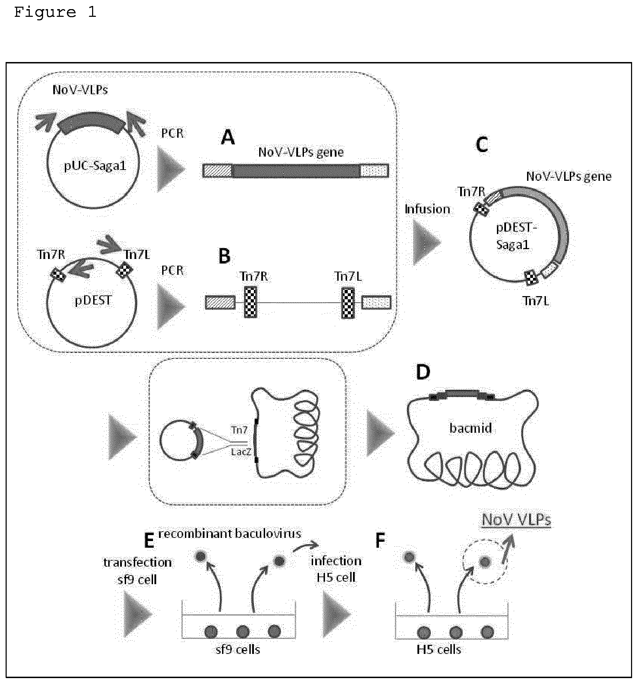

Artificial synthesis of DNAs encoding the VP1 and VP2 regions, which are structural protein regions, of the GII.4 Saga1 strain (Genbank No: AB447456), the GII.3 TCH strain (Genbank No. KF006265), and the GII.17 Saitama strain (Genbank No. KJ196286.1) of norovirus (hereinafter, may be abbreviated to NoV), was outsourced to Fasmac Co., Ltd., and a target gene was introduced therein using the position of the slash of 5'-CAGACGTGTGCTCTTCCGATCTGAT/ATCAGATCGGAAGAGCGTCGTTAAG-3' (SEQ ID NO: 270), which is the lacZ-**α** region of pUCFa plasmid, as the cloning site. In GII.4, a PCR reaction was performed using a synthesized pUC-Saga1 (Figure 1) DNA as a template and using primer 1 (5'-CATCACAAGTTTGTACAAAAAAGCAGGCTGTGA-3': SEQ ID NO: 271) and primer 2 (5'-TATCACCACTTTGTACAAGAAAGCTGGGTT-3': SEQ ID NO: 272) to obtain a fragment (Figure 1-A). In GII.3, a PCR reaction was performed using a synthesized pUC-TCH DNA as a template and using primer 3 (5'-ATCACAAGTTTGTACTGGGAGGGCGATCGCA-3': SEQ ID NO: 273) and primer 4 (5'-CTATCACCACTTTGTTCGCTACCTCGCGAA-3': SEQ ID NO: 274) to obtain a fragment (Figure 1-A). In addition, a PCR reaction was performed using a pDEST8 plasmid (Invitrogen) as a template and using primer 5 (5'-ACAAGTGGTGATAGCTTGTCGAGAAGTA-3': SEQ ID NO: 275) and primer 6 (5'-GTACAAACTTGTGATGATCCGCGCCCGAT-3': SEQ ID NO: 276) to obtain a fragment (Figure 1-B). The resulting PCR fragment A and fragment B were mixed and were reacted to each other using an InFusion HD Cloning Kit (Clontech Laboratories, Inc.), and using 1 ng of the resulting DNA (Figure 1-C), Competent Quick DH5**α** (manufactured by TOYOBO CO., LTD.) was transformed. Selection was performed with an LB agar plate culture medium containing 100 **µ**g/mL of ampicillin, the resulting colonies were cultured in an LB liquid culture medium containing 100 (**µ**g/mL of ampicillin, and the plasmid was extracted from the resulting cells using a QIAprep Spin Miniprep Kit (manufactured by QIAGEN N.V.). The sequence of the resulting plasmid with NoV gene introduced was determined using a DNA sequencer to verify that the target sequence was inserted.

In GII.17, a sequence in which an attL1 sequence (5'-ccccaaataatgattttattttgactgatagtgacctgttcgttgcaacaaattgat gagcaatgcttttttataatgccaactttgtacaaaaaagcaggct-3': SEQ ID NO: 277) was introduced to 4 nucleotides upstream from the start codon of the VP1 region and a polyadenine sequence of 30 adenines followed by an attL2 sequence (5'-agcttacccagctttcttgtacaaagttggcattataagaaagcattgcttatcaat ttgttgcaacgaacaggtcactatcagtcaaaataaaatcattatttg-3': SEQ ID NO: 278) were introduced to 55 nucleotides downstream from the termination codon of the VP2 region was artificially synthesized. The resulting pUC-Saitama and pDEST8 were mixed in equal amounts, and were mixed as shown in Table 1, followed by a reaction at 25°C for 1 hour. After the reaction, 1 **µ**L of proteinase K (manufactured by Takara Bio Inc.) was added thereto, followed by a reaction at 37°C for 10 minutes. Using 1 ng of the reaction solution, Competent Quick DH5**α** (manufactured by TOYOBO CO., LTD.) was transformed, and selection was performed with an LB agar plate culture medium containing 100 **µ**g/mL of ampicillin. The resulting colonies were cultured in an LB liquid culture medium containing 100 **µ**g/mL of ampicillin, and pDEST8 encoding GII.17 was purified and obtained from the resulting cells using a QIAprep Spin Miniprep Kit (manufactured by QIAGEN N.V.) .

**[Table 1]**

| Composition | Content |
|---|---|
| pUC-Saitama | 0.75 **µ**L (150ng) |
| pDEST8 (manufactured by Invitrogen) | 1.0 **µ**L (150ng) |
| UltraPure Water (manufactured by Invitrogen) | 6.25 **µ**L |
| BP clonase (manufactured by Thermo Fisher Scientific) | 2 **µ**L |

### (2) Introduction of VP1 and VP2 genes into bMON14272 Bacmid (manufactured by Invitrogen)

The obtained plasmid was introduced into Bacmid according to the protocol attached to the product by the following method (Figure 1-D).

NoV VP1 and VP2 regions were introduced into bMON14272 bacmid (manufactured by Invitrogen) using the obtained plasmid having Nov gene introduced and MAX Efficiency DH10Bac Competent Cells (manufactured by Invitrogen) (Figure 1-D). Whether each gene was introduced into bacmid or not was verified by performing selection in an LB culture medium containing 40 **µ**g/mL of IPTG (isopropyl **β**-D-1-thiogalactopyranoside: manufactured by FUJIFILM Wako Pure Chemical Corporation), 100 **µ**g/mL of X-Gal (5-bromo-4-chloro-3-indolyl **β**-D-galactopyranoside: manufactured by FUJIFILM Wako Pure Chemical Corporation), 50 **µ**g/mL of kanamycin, 7 **µ**g/mL of gentamicin, and 10 **µ**g/mL of tetracycline (kanamycin resistance gene, tetracycline resistance gene, and gentamicin resistance gene were encoded in bMON14272, helper plasmid present in DH10Bac Competent Cell, and the region of pDEST to be inserted into bacmid, respectively), and further whether each fragment was inserted into a target side or not was verified by color selection. The obtained white colonies were cultured in an LB liquid culture medium containing 50 **µ**g/mL of kanamycin, 7 **µ**g/mL of gentamicin, and 10 **µ**g/mL of tetracycline, and bacmid was extracted from the resulting cells using a QIAprep Spin Miniprep Kit (manufacture by QIAGEN N.V.). The concentration of the extracted DNA solution was verified with NanoDrop (manufactured by Thermo Fisher Scientific).

### (3) Production of recombinant baculovirus (rBV) by transfection of bacmid having NoV VP1 and VP2 introduced

Bacmid into which NoV VP1 and VP2 genes were introduced was transfected into Sf9 cells (manufactured by Invitrogen) using a Lipofecctamine LTX Reagent & Plus Reagent (manufactured by Invitrogen) according to the protocol attached thereto (Figure 1-E). The transfected cells were cultured using an Sf900III (manufactured by Invitrogen) culture medium at 27°C for 1 week. After the culture, the culture medium was centrifuged, and the supernatant was collected to obtain recombinant baculovirus (rBV) including the NoV gene.

### (4) Production of NoV VLP by infection with rBV

The rBV was added at 1.0 × 10⁷ pfu/mL to 1.0 × 10⁷ cells/flask of High Five cells (manufactured by Invitrogen) to cause infection at an MOI of 2, and the cells were cultured using an Express five (manufactured by Invitrogen) culture medium at 27°C (Figure 1-F). After 7 days from the infection, the culture supernatant was collected by centrifugation. The collected supernatant was further centrifuged at 10,000 × g for 1 hour to pellet down the baculovirus, and the cell supernatant was collected. The collected supernatant containing NoV VLP was further centrifuged with an SW32Ti rotor (manufactured by Beckman Coulter, Inc.) at 32,000 rpm for 2 hours to pellet down the NoV VLP. The pellet separated from the supernatant was dissolved in an Express five culture medium containing 1.9 mg of CsCl (for density gradient centrifugation, manufactured by FUJIFILM Wako Pure Chemical Corporation) and centrifuged using SW55Ti (Manufactured by Beckman Coulter, Inc.) an at 40,000 rpm for 20 hours for separation and purification, and a fraction visually observed by irradiation with white light was collected. The collected fraction was centrifuged again with the SW32Ti rotor at 32,000 rpm for pellet down, the supernatant was removed, and the pellet was suspended in 500 **µ**L of an Express five culture medium. The VLP concentration was quantitatively measured by a Bradford method. As a standard protein, BSA (manufactured by FUJIFILM Wako Pure Chemical Corporation) was used.

### Example 1: Manufacturing of norovirus-binding peptide (see Figure 2)

### (1) Construction of DNA library

A DNA library was designed such that the peptide library is composed of peptides having a length of 10 amino acids.

### (Nucleotide sequence of DNA library)

5'-

GATCCCGCGAAATTAATACGACTCACTATAGGGGAAGTATTTTTACAACAATTACCA

ACAACAACAACAAACAACAACAACATTACATTTTACATTCTACAACTACAAGCCACC

ATGGGCAGCGGCGGCAGCTGCNNKNNKNNKNNKNNKNNKTGCGGGGGAGGCAGCCAT

CATCATCATCATCACGGCGGAAGCAGGACGGGGGCGGCGTGGAAA-3' (SEQ ID

NO: 279)

**[Table 2]**

| Each sequence included in template DNA and position thereof | | |
|---|---|---|
| Nucleotide No. | | Region Name |
| 14 to 33 | | T7 promoter |
| 34 to 36 | | 5' cap |
| 37 to 107 | | **Ω** sequence |
| 110 to 114 | | Kozak sequence |
| 115 to 132 | | MGSGGS |
| 133 to 156 | | Cys-library sequence-Cys |
| 157 to 168 | | GGGS |
| 169 to 186 | | Hexahistidine tag |
| 187 to 195 | | GGS |
| 196 to 217 | | Hybridization region for linker DNA |

The above-mentioned DNA library was constructed by binding three DNA sequence fragments, a T7-PRO-**Ω** region (SEQ ID NO: 280), a random region (SEQ ID NO: 281), and a His-Y tag region (SEQ ID NO: 282), by extension PCR. This library was designed such that cysteines appear on the N-terminus and the C-terminus of the random region. The random region, the His-Y tag region, and the T7-PRO-**Ω** region were obtained by outsourcing the respective DNA synthesis to TSUKUBA OLIGO SERVICE CO. LTD. The following extension PCR was performed using them to construct the above DNA library.

### [SEQ ID NO: 280]

5'

GATCCCGCGAAATTAATACGACTCACTATAGGGGAAGTATTTTTACAACAATTACCA

ACAACAACAACAAACAACAACAACATTACATTTTACATTCTACAACTACAAGCCACC

ATG 3'

### [SEQ ID NO: 281]

5'-
ACAACTACAAGCCACCATGGGCAGCGGCAGCTGCNNKNNKNNKNNKNNKNNKTG
CGGGGGAGGCAGCCATCATCA-3'

In the sequences above, the ratios of appearance frequency of nucleotides (A : T : G : C) other than ATCG are as follows:
N: A = 0.25, T = 0.25, G = 0.25, C = 0.25, and
K: A = 0, T = 0.5, G = 0.5, C = 0.

### [SEQ ID NO: 282]

5'
TTTCCACGCCGCCCCCCGTCCTGCTTCCGCCGTGATGATGATGATGATGGCTGCCTC
CCCC 3'

In the extension PCR of the first stage of synthesis, a reaction solution having the composition shown in the following Table 3 was prepared to 50 **µ**L with ultrapure water, and a DNA fragment including the random region and the His-Y tag region bound to each other was amplified by the following PCR program. The PCR program included (a) 96°C (2 min), (b) 94°C (20 sec), (c) 69°C (5 sec), (d) 72°C (20 sec), and (e) 72°C (2 min), and the steps (b) to (d) were repeated 5 cycles.

**[Table 3]**

| | Composition | Content (**µ**L) |
|---|---|---|
| Random region (10 pmol/ **µ**L) | | 1 |
| His-Y tag region (10 pmol/ **µ**L) | | 1 |
| 5 x PrimeSTAR Buffer (manufactured by Takara Bio Inc.) | | 10 |
| dNTP mixture (25 mM each) (manufactured by Takara Bio Inc.) | | 4 |
| TaKaRa PrimseSTAR (manufactured by Takara Bio Inc.) | | 0.5 |

In the extension PCR of the second stage, a reaction solution having the composition shown in the following Table 4 was prepared to 50 **µ**L with ultrapure water, the T7-PRO-SD region was extended by the following PCR program to amplify the DNA library. The PCR program included (a) 96°C (2 min), (b) 94°C (20 sec), (c) 59°C (5 sec), (d) 72°C (30 sec), and (e) 72°C (2 min), and the steps (b) to (d) were repeated 15 cycles. Subsequently, the DNA library was purified by polyacrylamide gel electrophoresis (PAGE).

**[Table 4]**

| | Composition | Content (**µ**L) |
|---|---|---|
| Extended PCR product in first stage (0.5 pmol/**µ**L) | | 10 |
| T7-PRO-**Ω** region (10 pmol/**µ**L) | | 5 |
| 5 × PrimeSTAR Buffer (manufactured by Takara Bio Inc.) | | 10 |
| dNTP mixture (25 mM each) (manufactured by Takara Bio Inc.) | | 4 |
| TaKaRa PrimeSTAR (manufactured by Takara Bio Inc.) | | 0.25 |

### (2) Transcription of DNA library

Transcription of the DNA library was performed using RiboMAX Large Scale RNA Production Systems-T7 (manufactured by Promega Corporation) according to the protocol attached thereto. The reaction scale was 20 **µ**L using 1 **µ**g of the DNA library. The mRNA obtained by the transcription reaction was purified using an After Tri-Reagent RNA Clean-Up Kit (manufactured by FAVORGEN Biotech Corporation).

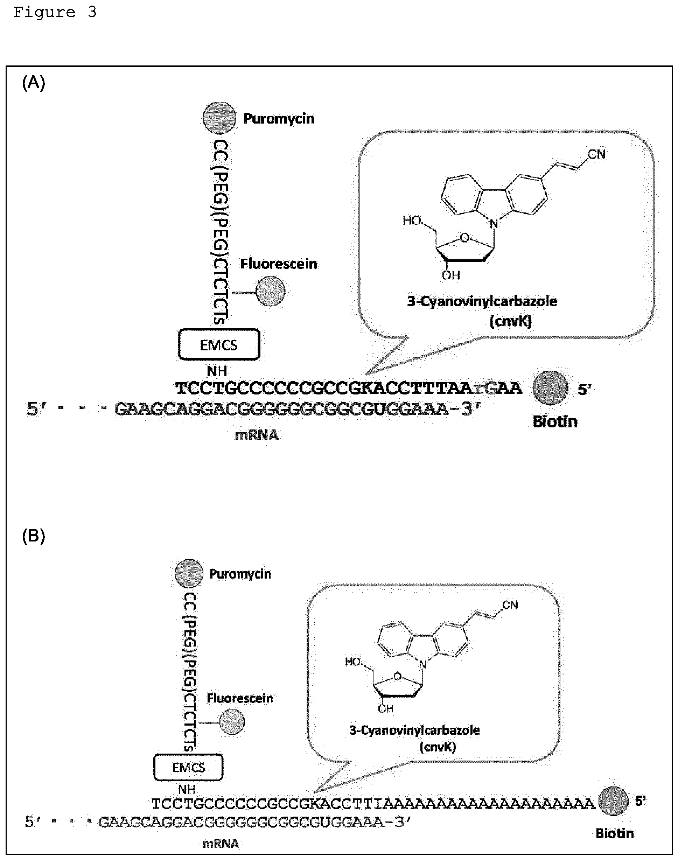

Subsequently, the obtained mRNA was ligated to a puromycin linker described later as follows (Figure 3 (A)). Firstly, 20 pmol of each of a puromycin linker and the mRNA, 4 **µ**L of 0.25 M Tris-HCl (pH 7.5), and 4 **µ**L of 1 M NaCl were mixed, and the mixture was diluted to 20 **µ**L with ultrapure water. The reaction solution was incubated at 90°C for 2 minutes and at 70°C for 1 minute, was then cooled to 4°C, and was then annealed at 25°C for 1 hour. Subsequently, crosslinking with the puromycin linker was performed using a CL-1000 Ultraviolet Crosslinker by irradiation with ultraviolet light having a wavelength of 365 nm under a condition of 405 mJ/cm².

### <DNA of puromycin linker>

Puromycin linker DNA 1 (Figure 3 (A)) was synthesized by chemical crosslinking of two segments (puromycin segment (PS) and a short biotin segment (SBS)) using EMCS (N-(6-maleimidocaproyloxy)succinimide: manufactured by DOJINDO LABORATORIES). The linker used was that described in the literature (Mochizuki Y., Suzuki T., Fujimoto K., Nemoto N., (2015), A versatile puromycin-linker using cnvK for high-throughput in vitro selection by cDNA display, J. Biotechnol., 212, 174-80).

The sequence structure of the puromycin segment (PS) is shown below:
5'-(S)-TCTCTC(F)-(PEG)(PEG)-CC-(Puro)-3'. Here, (S) represents 5'-thiol-modifier C6 (compound name: S-trityl-6-mercaptohexyl-1-[(2-cyanoethyl)-(N,N-diisopropyl)]-phosphoramidite, manufactured by Glen research), and (F) represents fluoresceine-dT. (Puro) represents puromycin CPG (5'-dimethoxytrityl-N-trifluoroacetyl-puromycin,2'-succinoyl-long chain alkylamino-CPG, manufactured by Glen research).

The sequence structure of the short biotin segment (SBS) is then shown below:
5'(B)-AA-(rG)-AATTTCCA(K)GCCGCCCCCCG(Y)CCT-3' (SEQ ID NO: 283).

Here, (Y) represents amino-modifier C6 deoxythymine (5'-dimethoxytrityl-5-[N-(trifluoroacetylaminohexyl)-3-acrylimido]-2'-deoxyuridine,3'-[(2-cyanoethyl)-(N,N-diisopropyl)]-phosphoramidite, manufactured by Glen research), (K) represents 3-cyanovinylcarbazole (cnvK), (B) represents biotin-triethylene glycol (TEG), manufactured by Glen research), and (rG) represents riboguanine (manufactured by Glen research). Synthesis of PS and SBS was outsourced to TSUKUBA OLIGO SERVICE CO. LTD. and was performed according to a usual method.

The 5'-thiol group of the PS was reduced with 1 mM tris(2-carboxyethyl)phosphine hydrochloride (TCEP: manufactured by Thermo Fisher Scientific) in 100 **µ**L of a 50 mM phosphate buffer (pH 7.0) at room temperature for 6 hours, and desalted with a NAP-5 column (manufactured by GE Healthcare) at the time of use. A biotin loop in a total amount of 10 nmol and EMCS in a total amount of 2 **µ**mol were added to 100 **µ**L of a 0.2 M sodium phosphate buffer (pH 7.0). Subsequently, the mixture was incubated at 37°C for 30 minutes, and ethanol precipitation was performed at 4°C to remove excess EMCS.

This precipitate was washed twice with 500 **µ**L of 70% ethanol cooled in advance in an ice bath and was dissolved in 10 **µ**L of a 0.2 M sodium phosphate buffer (pH 7.0) cooled in advance. The reduced PS was immediately added thereto, followed by stirring at 4°C overnight. After addition of 4 mM TCEP, incubation was performed at 37°C for 15 minutes to stop this reaction. Subsequently, ethanol precipitation was performed to remove excess PS at room temperature. In order to remove the biotin loop and uncrosslinked biotin loop-EMCS complex, the precipitate was dissolved in a 0.1 M TEAA (triethylamine acetate: manufactured by Glen research) or phosphate buffer and was purified using a C18 HPLC column under the following condition:

Column: AR-300, 4.6 × 250 mm (manufactured by NACALAI TESQUE, INC.); solvent: A: 0.1 M TEAA, solvent B: acetonitrile/water (80 : 20, v/v) gradient, B/A (15 to 35%, 33 min); flow rate: 0.5 mL/min; and detection wavelength: absorbance 254 nm and 490 nm.

Fractions from the final peak at absorbance 254 nm (corresponding to a single peak at absorbance 490 nm) were collected. After drying, the fractions were resuspended in water treated with diethylpyrocarbonate (DEPC) and were stored. As described above, puromycin linker DNA 1 could be obtained.

### <Binding of mRNA and puromycin linker DNA 1>

To 20 pmol of the mRNA obtained by transcription, 20 pmol of the puromycin linker DNA 1, 4 **µ**L of 0.25 M Tris-HCl (pH 7.5), and 4 **µ**L of 1 M NaCl were added, and prepared to 20 **µ**L in total with nuclease-free water (Table 5). Incubation was performed at 90°C for 1 minute and then at 70°C for 1 minute, and the temperature was then lowered to 25°C at a rate of 0.04°C/s. The cnvK and uracil in the mRNA were covalently bonded by irradiation with 405 mJ of ultraviolet light (365 nm) to form a mRNA-linker conjugate. The amount synthesized here was that required in each round.

**[Table 5]**

| Composition for binding of mRNA and puromycin linker DNA | | |
|---|---|---|
| | Composition | Content |
| Puromycin linker DNA 1 | | 20 pmol |
| mRNA | | 20 pmol |
| 0.25 M Tris-HCl (pH 7.5) | | 4 **µ**L |
| 1 M NaCl | | 4 **µ**L |

### <Translation>

The mRNA-linker conjugate was translated by a cell-free translation system as follows. A reaction solution having the composition ratio shown in the following Table 6 was prepared to 50 **µ**L with ultrapure water and was reacted at 37°C for 15 minutes, and 24 **µ**L of 3 M KCl and 6 **µ**L of 1 M MgCl₂ were added to this reaction solution. Subsequently, this solution was further reacted at 37°C for 20 minutes to bind between the C-terminus of the translated peptide and puromycin to obtain a mRNA-peptide conjugate.

**[Table 6]**

| | Composition | Content |
|---|---|---|
| Rabbit reticulocyte lysate, nuclease treated (manufactured by Promega Corporation) | | 35 **µ**L |
| Amino acid mixture minus leucine, 1 mM (manufactured by Promega Corporation) | | 0.5 **µ**L |
| Amino acid mixture minus cysteine, 1 mM (manufactured by Promega Corporation) | | 0.5 **µ**L |
| mRNA/linker ligation product | | 6 pmol |

### (3) Purification by magnetic beads

Streptavidin (SA) magnetic particles (Dynabeads MyOne Streptavidin C1, manufactured by Invitrogen) were washed according to the manual and were put in an Eppendorf tube in an amount required for immobilizing the peptide-linker-mRNA conjugate, followed by leaving to stand on a magnetic stand for 1 minute. Subsequently, the supernatant was removed, followed by resuspension in a solution A (100 mM NaOH, 50 mM NaCl). After tapping for 1 to 2 minutes, the tube was left to stand on a magnetic stand for 1 minute. Subsequently, the same operation was repeated once with the solution A, and the same operation was repeated once with a solution B (100 mM NaCl).

To the peptide-linker-mRNA conjugate, the same amount of 2 × binding buffer (20 mM Tris-HCl (pH 8.0), 2 mM EDTA, 2 M NaCl, 0.2% Tween 20, and 500 mM EDTA) was added, and the mixture was incubated together with the streptavidin (SA) magnetic particles at room temperature for 30 minutes. The Eppendorf tube was left to stand on a magnetic stand for 1 minute, and the supernatant was then removed. After addition of 200 **µ**L of 1 × binding buffer, tapping was performed for 1 to 2 minutes, and the tube was then left to stand on a magnetic stand for 1 minute, followed by removal of the supernatant. This operation was further repeated twice to immobilize the peptide-linker-mRNA conjugate on the streptavidin (SA) magnetic particles.

### (4) Synthesis of cDNA by reverse transcription reaction

A reaction solution of the ratio shown in the following Table 7 was added to the immobilized peptide-linker-mRNA conjugate in the same volume as that of the streptavidin (SA) magnetic particles, and incubation was performed at 42°C for 30 minutes for reverse transcription to prepare cDNA display in the state that the conjugate was immobilized on the streptavidin (SA) magnetic particles.

**[Table 7]**

| | Composition | Content (**µ**L) |
|---|---|---|
| 2.5 mM dNTP MIX (manufactured by Takara Bio Inc.) | | 20 |
| 5 x RT Buffer (manufactured by TOYOBO CO., LTD.) | | 10 |
| Nuclease-free water | | 18 |
| ReverTra Ace (manufactured by TOYOBO CO., LTD.) | | 2 |

### (5) Crosslinking reaction of peptide

The cDNA display immobilized on the streptavidin (SA) magnetic beads were washed with a crosslinking buffer (containing 100 mM sodium phosphate (pH 7.4), 0.15 M NaCl, 10 mM EDTA, and 0.025% Tween 20) once, and then 125 **µ**L of a crosslinking buffer containing 10 mM tris(2-carboxyethyl)phosphine hydrochloride (TCEP: manufactured by Thermo Fisher Scientific) and 4 mM bismaleimidoethane (BMOE: manufactured by Thermo Fisher Scientific) was added, followed by incubation at 25°C for 1 hour to perform crosslinking reaction of the cysteines on the N-terminus and the C-terminus of the random region.

### (6) Elution from purification by magnetic beads

The cDNA display immobilized on the streptavidin (SA) magnetic beads was washed with 1 × His-tag wash buffer (containing 10 to 30 mM sodium phosphate (pH 7.4), 0.25 to 0.75 M NaCl, 5 to 30 mM imidazole, and 0.025% to 0.1% Tween 20) once, and then 30 **µ**L of 1 x His-tag wash buffer containing 10 U of RNase T1 (manufactured by Ambion, Inc.) was added, followed by incubation at 37°C for 15 minutes to elute the cDNA display cleaved from the streptavidin (SA) magnetic beads at the cleavage site (ribo G) in the liker.

### (7) Purification by Ni-NTA

Ni-NTA magnetic beads (His Mag Sepharose Ni: manufactured by GE Healthcare) were put at 10 **µ**L in an Eppendorf tube, followed by leaving to stand on a magnetic stand for 1 minute. Subsequently, the supernatant was removed, followed by resuspension in 1 × His-tag wash buffer. Tapping was performed for 1 to 2 minutes, and the tube was then left to stand on a magnetic stand for 1 minute. This procedure was further repeated once more.

The cDNA display was incubated together with the Ni-NTA magnetic beads at room temperature for 30 minutes. The Eppendorf tube was left to stand on a magnetic stand for 1 minute, and the supernatant was then removed. After addition of 200 **µ**L of 1 × His-tag wash buffer, tapping was performed for 1 to 2 minutes, and the tube was then left to stand on a magnetic stand for 1 minute, followed by removal of the supernatant. This operation was further repeated, and 10 **µ**L of a His-tag elution buffer (containing 10 to 30 mM sodium phosphate (pH 7.4), 0.25 to 0.75 M NaCl, 250 to 500 mM imidazole, and 0.025% to 0.1% Tween 20) was then added, followed by incubation at room temperature for 15 minutes to purify the cDNA display.

### (8) In vitro selection cycle

The cDNA displays and VLPs were mixed according to the following Table 8 and were prepared to 1 mL with a selection buffer (containing 10 to 30 mM Tris-HCl (pH 7.4) and 0.1 to 0.3 M NaCl), followed by incubation at 25°C for 30 minutes.

**[Table 8]**

| | cDNA display | VLP |
|---|---|---|
| R1 | 100 nM | GII.4 500 nM |
| R2 | 16 nM | GII.4 50 nM |
| R3 | 10 nM | GII.4 25 nM |
| R4 | 4 nM | GII.4 25 nM |
| R5 | 4 nM | GII.4 20 nM |
| R6 | 4 nM | GII.4 10 nM |
| R7 | 4 nM | GII.4 10 nM |

R1 to R7 are the numbers of in vitro selection cycles.

### <Separation of VLP-cDNA display complex by centrifugation>

The above mixture was put in a centrifuge tube and was centrifuged with an ultracentrifuge (CS150FNX, manufactured by Hitachi, Ltd.) at 130,000 x g at 4°C for about 2 hours. The supernatant was removed. The wall surface was washed with 1 mL of a selection buffer, and the supernatant was then removed. The precipitate was redissolved in 100 **µ**L of RNase-free water.

### <Separation of VLP-cDNA display complex by dialysis>

The constructed cDNA display and VLPs were incubated in 100 **µ**L of a dialysis selection buffer (20 mM HEPES (pH 7.4), 150 mM NaCl, and 0.05% Tween 20) at the concentrations shown in Table 9 below at 25°C for 30 minutes. Subsequently, the resultant was diluted to 1 mL with the dialysis selection buffer, and was put in Float-A-Lyzer G2 Dialysis Device CE, 1000kD MWCO (manufactured by Spectrum Laboratories, Inc.) and was dialyzed with 1 L of the dialysis selection buffer as the external solution at 25°C. During the dialysis, the external solution was replaced with new one 3 times every 2 hours, and the dialysis was performed overnight (for 8 hours) after the 4th replacement. The dialysis product was concentrated with Amicon Ultra 100K (manufactured by Merck Millipore S.A.S.) at 14,000 × g for 5 minutes.

### (9) Selection of GII.3 and GII.17 VLP as objects

To an immunoplate (C-BOTTOM, CLEAR, MICROLON (registered trademark), HIGH BINDING, manufactured by Greiner Bio-One), 100 **µ**L of 3 **µ**g/mL GII.3 or GII.17 VLP solution was added and immobilization was performed at 4°C overnight. Subsequently, the solution was discarded, and 200 **µ**L of a blocking agent (EMD Millipore^{™} Blok^{™} NSB Blocking agents, Thermo Fisher Scientific) was added, followed by gently stirring at room temperature for 2 hours for blocking. On this occasion, wells not immobilizing the VLP were also subjected to similar blocking to be used in preselection.

The solution was discarded from the wells, the wells were washed with 200 **µ**L of a wash buffer (10 mM Hepes (pH 7.4), 150 mM NaCl, and 0.05% Tween 20) three times, and 100 **µ**L of cDNA display (constructed from 1.5 pmol of mRNA-linker) was then put in the wells not immobilizing the VLP, followed by gently stirring at room temperature for 30 minutes to perform preselection. Subsequently, the supernatant containing cDNA display that had not bound to the blocking agent was put in the wells immobilizing the VLP, followed by gently stirring at room temperature for 30 minutes to be bound to the VLP. The supernatant was discarded, washing with 100 **µ**L of the wash buffer was performed four times, and 100 **µ**L of a 5% SDS solution was then added, followed by incubation at 50°C for 15 minutes to elute the bound cDNA display.

Subsequently, the VLP-cDNA display complex obtained above was diluted to 100 **µ**L with a dialysis selection buffer, and 10 **µ**L of a coprecipitating agent (Quick-Precip Plus Solution, manufactured by EdgeBio) and 220 **µ**L of 100% ethanol were added, followed by centrifugation at 20,000 × g for 5 minutes. Subsequently, the supernatant was discarded, and 1 mL of 70% ethanol was added for rinsing. The tube was dried for 10 minutes, elution with 20 **µ**L of RNase-free water was then performed, and PCR reaction was performed using GATCCCGCGAAATTAATACGACTCACTATAGGGGAAGTATTTTTACAACAATTACCA ACA (SEQ ID NO: 284) as a forward primer and TTTCCACGCCGCCCCCCGTCCT (SEQ ID NO: 285) as a reverse primer. The PCR program was (a) 98°C for 2 minutes, (b) 95°C for 20 seconds, (c) 69°C for 20 seconds, (d) 72°C for 20 seconds (steps (b) to (d) were performed 25 cycles), and (e) 72°C for 1 minute.

**[Table 9]**

| | Composition | Content (**µ**L) |
|---|---|---|
| 10 × Ex Taq Buffer (manufactured by Takara Bio Inc.) | | 2.5 |
| 2.5 mM dNTP mixture (manufactured by Takara Bio Inc.) | | 2 |
| 20 **µ**M forward primer (SEQ ID NO: 284) | | 0.5 |
| 20 **µ**M reverse primer (SEQ ID NO: 285) | | 0.5 |
| Ethanol precipitate | | 3 |
| Nuclease-free water | | 16.4 |
| Ex Taq | | 0.1 |

The resulting PCR product was used as library DNA in the subsequent cycle, and the operations after the transcription of library described in the above (2) were similarly performed to repeat a selection cycle.

### <Analysis of genetic sequence information>

After the in vitro selection cycle (7 cycles for GII.4 and 5 cycles for GII.3 and GII.17 based on the libraries of 7 cycles obtained by GII.4 dialysis), a sequence library was prepared by the following method, and the sequence information was analyzed. The preparation of the sequence library and the sequencing were performed according to the 16S Metagenomic Sequencing Library Preparation protocol (manufactured by Illumina, Inc.).

### 1) Amplicon PCR

The reagents shown in Table 10 were mixed, and PCR was performed by the following program:
at 95°C for 3 minutes;
23 cycles of the following reactions;
   at 95°C for 30 seconds,
   at 55°C for 30 seconds, and
   at 72°C for 30 seconds,
at 72°C for 5 minutes; and holding at 4°C.

**[Table 10]**

| Composition of solution | |
|---|---|
| Sequence library (5 ng/**µ**L) | 2.5 **µ**L |
| Amplicon PCR Forward Primer 1 **µ**M (SEQ ID NO: 286) | 5 **µ**L |
| Amplicon PCR Reverse Primer 1 **µ**M (SEQ ID NO: 287) | 5 **µ**L |
| 2 × KAPA HiFi Hotstart ReadyMix (manufactured by NIPPON Genetics Co., Ltd. ) | 12.5 **µ**L |
| Total | 25 **µ**L |

### SEQ ID NO: 286:

TCGTCGGCAGCGTCAGATGTGTATAAGAGACAGCATTCTACAACTACAAGCCACCAT
G

### SEQ ID NO: 287:

GTCTCGTGGGCTCGGAGATGTGTATAAGAGACAGTTTCCACGCCGCCCCCCGTCCTG
CTTC

### 2) Clean up

The Amplicon PCR product was purified using AMPure XP beads (manufactured by Beckman Coulter, Inc.). To the plate including the PCR product, 20 **µ**L of the AMPure XP beads were added and the mixture was gently mixed by pipetting with a micropipette 10 times, followed by leaving to stand at room temperature for 5 minutes. The plate was placed on a magnetic stand and was left to stand for 2 minutes, and the supernatant was then discarded. While the plate was being placed on the magnetic stand, 200 **µ**L of 80% ethanol was added to each well, and after leaving to stand for 30 seconds, the supernatant was discarded. This procedure was repeated twice. The ethanol was air-dried by leaving to stand for 10 minutes, the plate was then taken out from the magnetic stand, and 52.5 **µ**L of 10 mM Tris pH 8.5 solution was added to each well, followed by leaving to stand at room temperature for 2 minutes. The plate was placed on the magnetic stand and was left to stand for 2 minutes again, and 50 **µ**L of the solution in each well was transferred to the corresponding well of a new 96-well PCR plate.

### 3) Index PCR

A PCR reaction was performed for adding an adaptor and an index sequence for sequencing to the purified Amplicon PCR product.

The reagents shown in Table 11 were mixed, and PCR was performed by the following program:
at 95°C for 3 minutes;
8 cycles of the following reactions;
   at 95°C for 30 seconds,
   at 55°C for 30 seconds, and
   at 72°C for 30 seconds,
at 72°C for 5 minutes; and holding at 4°C.

**[Table 11]**

| Composition of solution | |
|---|---|
| Purified Amplicon PCR product | 2.5 **µ**L |
| Nextera XT Index Primer 1 (N7xx) (SEQ ID NOs: 288 and 289) | 2.5 **µ**L |
| Nextera XT Index Primer 2 (S5xx) (SEQ ID NOs: 290 and 291) | 2.5 **µ**L |
| 2x KAPA HiFi Hotstart ReadyMix | 12.5 **µ**L |
| UltraPure Water (manufactured by Invitrogen) | 5 **µ**L |
| Total | 25 **µ**L |

The used Index Primer set is shown in the following Table 12.

**[Table 12] Index Primer set**

| | Centrifugation | Dialysis |
|---|---|---|
| Run 1 (S511) | S511, N724 | S511, N728 |
| Run 2 (S510) | S510, N724 | S510, N728 |

SEQ ID NO: 288 (N724):
CAAGCAGAAGACGGCATACGAGATACTGAGCGGTCTCGTGGGCTCGG

SEQ ID NO: 289 (N728):
CAAGCAGAAGACGGCATACGAGATTGCAGCTAGTCTCGTGGGCTCGG

SEQ ID NO: 290 (S511):
AATGATACGGCGACCACCGAGATCTACACTCTCTCCGTCGTCGGCAGCGTC

SEQ ID NO: 291 (S510):
AATGATACGGCGACCACCGAGATCTACACCGTCTAATTCGTCGGCAGCGTC

### 4) Clean up 2

The Index PCR product was purified using AMPure XP beads (manufactured by Beckman Coulter, Inc.). To the plate including the PCR product, 56 **µ**L of the AMPure XP beads were added and the mixture was gently mixed by pipetting with a micropipette 10 times, followed by leaving to stand at room temperature for 5 minutes. The plate was placed on a magnetic stand and was left to stand for 2 minutes, and the supernatant was then discarded. While the plate was being placed on the magnetic stand, 200 **µ**L of 80% ethanol was added to each well, and after leaving to stand for 30 seconds, the supernatant was discarded. This procedure was repeated twice. The ethanol was air-dried by leaving to stand for 10 minutes, the plate was then taken out from the magnetic stand, and 25 **µ**L of 10 mM Tris pH 8.5 solution was added to each well, followed by leaving to stand at room temperature for 2 minutes. The plate was placed on the magnetic stand and was left to stand for 2 minutes again, and 50 **µ**L of the solution in each well was transferred to the corresponding well of a new 96-well PCR plate.

The purified Index PCR product was validated using Bioanalyzer DNA 1000 Chip (manufactured by Agilent Technologies, Inc.).

### 5) qPCR

The purified Index PCR product was subjected to qPCR using Kapa Library Quantification Kit (manufactured by NIPPON Genetics Co., Ltd.).

A mixture of 12 **µ**L of Kapa SYBR FAST qPCR Master Mix to which Primer Mix was added in advance, 4 **µ**L of UltraPure Water, and 4 **µ**L of a 100-fold dilution of the Index PCR product was subjected to qPCR. As the samples for a standard curve, Std 1 to 6 included in the kit were used.

The PCR was performed by the following program:
at 95°C for 5 minutes; and
35 cycles of the following reactions;
   at 95°C for 30 seconds, and
   at 60°C for 45 seconds.

A standard curve was drawn from the Ct values of Std 1 to 6, and sample concentration was calculated.

### 6) Preparing DNA Libraries for Sequencing

The Reagent Cartridge of Miseq Reagent Kit V3 150 cycles (manufactured by Illumina, Inc.) was thawed in a water bath, and the HT1 buffer included in the kit was thawed at room temperature and ice-cooled.

The Index PCR product having a concentration known by qPCR was diluted to 4 nM with UltraPure Water. A mixture of 5 **µ**L of this 4 nM dilution of the sample and 5 **µ**L of 0.2 N NaOH (prepared by diluting 10 N NaOH aqueous solution (manufactured by FUJIFILM Wako Pure Chemical Corporation) to 0.2 N with UltraPure Water) was left to stand at room temperature for 5 minutes. Subsequently, 990 **µ**L of ice-cooled HT1 buffer was added thereto to obtain 1 mL of a 20 pM denatured library.

A mixture of 180 **µ**L of the 20 pM denatured library and 420 **µ**L of ice-cooled HT1 buffer was prepared as 600 **µ**L of a 6 pM library. In addition, a mixture of 30 **µ**L of a 20 pM PhiX DNA denatured in advance and 10 **µ**L of ice-cooled HT1 buffer was prepared as a 15 pM denatured PhiX. A mixture of 30 **µ**L of the 15 pM denatured PhiX and 570 **µ**L of the 6 pM denatured library in total of 600 **µ**L was added to "Load Samples" (position 17) of the Reagent Cartridge thawed in a water bath.

### 7) Starting the Run

Flow Cell washed with Milli-Q water and 99.5% ethanol was set to Miseq (manufactured by Illumina, Inc.) subjected to Maintainance Wash with 0.5% Tween 20, and a PR2 bottle and a reagent-filled cartridge were set, followed by sequencing.

### 8) Analysis of gene information

The Fastq file of the obtained sequence was converted to a Fasta file, all of the obtained sequences were simultaneously translated from the first base of the start codon (ATG) at position 115 to the third base of the cysteine codon (TGC) at position 156 of the library sequence (SEQ ID NO: 279) using software MEGA. After the translation, the amino acid 7 residues upstream from the terminal cysteine was filtered with cysteine using the filter function of Excel to obtain 4357 peptide aptamer sequences.

### 9) Selection of sequence

The obtained 7069 peptide aptamer sequences were subjected to appearance frequency analysis, and the peptides shown in SEQ ID NOs: 1 to 269, which are sequences having an appearance frequency of 6 or more, were selected as peptides that specifically bind to norovirus. The selected 269 sequences are shown in Table 16.

### Example 2: Interaction with VLPs

### <Synthesis of peptide>

Peptides were synthesized by Fmoc solid synthesis in a nitrogen atmosphere using an automated peptide synthesizer Liberty Blue (manufactured by CEM Corporation). The resin used was Fmoc-Lys (Mtt)-Wang resin (manufactured by Merck Millipore S.A.S.) or Fmoc-Cys (Trt)-Wang Resin (manufactured by PEPTIDE INSTITUTE, INC.). N,N-Dimethylformamide: DMF (manufactured by FUJIFILM Wako Pure Chemical Corporation) was used as a solvent, and piperidine (manufactured by FUJIFILM Wako Pure Chemical Corporation) diluted with DMF to a predetermined concentration was used as a deprotecting agent. Diisopropylcarbodiimide (manufactured by Tokyo Chemical Industry Co., Ltd.) and ethyl cyanoglyoxylate-2-oxime: Oxyma (manufactured by WATANABE CHEMICAL INDUSTRIES, LTD.) diluted with DMF to predetermined concentrations were used as a coupling reaction accelerator and an optical activity inhibitor, respectively. The synthesis reaction was performed according to the synthesis program provided in the apparatus.

### <Introduction of biotin by manual synthesis>

A synthesis reaction was performed using a manual peptide synthesizer Petisyzer (manufactured by HiPep Laboratories). Biotin was introduced to the C-terminus side of the tryptophan by using a mixture solution prepared at the following ratio (Table 13) and stirring at room temperature for 1 hour. After the reaction, washing with DMF and diethyl ether was performed.

**[Table 13]**

| Reagent name | Equivalent |
|---|---|
| HBTU | 5 |
| HOBt | 4.5 |
| Biotin (manufactured by Tokyo Chemical Industry Co., Ltd.) | 5 |
| DIEA | 10 |

### <Cleavage of peptide attached with spacer sequence from resin>

The resin binding to the peptide added with a spacer sequence was washed with diethyl ether (manufactured by FUJIFILM Wako Pure Chemical Corporation) and dried. This resin was brought into contact with a mixture solution of TFA : TIS : H₂O = 95 : 2.5 : 2.5 at room temperature for 1 hour to cleave the peptide from the resin. The resin was removed from the solution by filtration, and 5 times the amount of ice-cooled diethyl ether was added to the solution, followed by inversion and stirring to generate a precipitate. The generated precipitate was centrifuged at 13,000 rpm for 3 minutes at 20°C, and the precipitate was again washed with diethyl ether and centrifuged under the same conditions. The precipitate was dried in a draft, and the resulting powder was stored at 4°C.

### <Evaluation of interaction by ELISA>

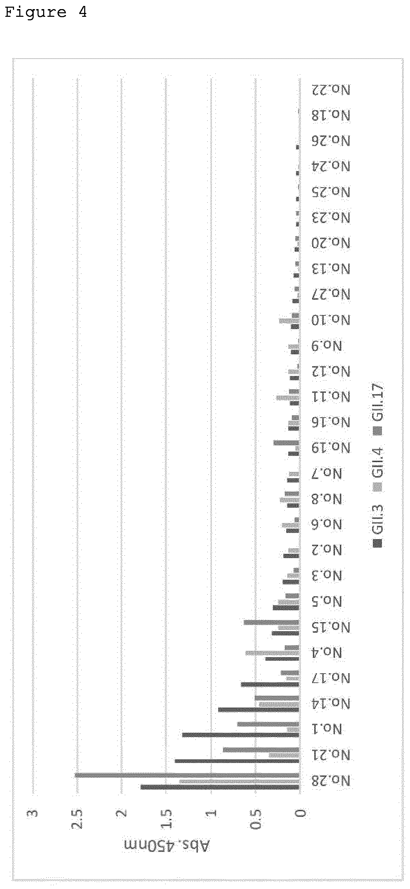

The peptide was dispersed in a 10% DMF aqueous solution. The concentration was calculated by an absorptiometer and was adjusted to 50 **µ**M with a 10% DMF aqueous solution. This was added to Pierce^{™} Streptavidin Coated Plates, Clear, 96-Well (manufactured by Thermo Fisher Scientific) at 100 **µ**L/well and was left to stand at room temperature for 1 hour. The supernatant was removed, and after washing with 200 **µ**L of PBS-T (PBS containing 0.05% Tween 20) three times, 100 **µ**L of a GII.3, GII.4, or GII.17 VLP solution diluted to 100 ng/mL with PBS-T was added, followed by leaving to stand at room temperature for 50 minutes. The supernatant was removed, and after washing with 200 **µ**L of PBS-T three times, 100 **µ**L of a rabbit anti-norovirus VLP polyclonal antibody (produced using a mixture of the GII.3 and GII.17 VLPs as an antigen by outsourcing to Eurofins Genomics K.K.) diluted to 1 **µ**g/mL with a blocking agent was added, followed by leaving to stand at room temperature for 50 minutes. The supernatant was removed, and after washing with 200 **µ**L of PBS-T three times, 100 **µ**L of an HRP-labeled anti-rabbit IgG antibody (manufactured by Cell Signaling Technology, Inc.) diluted 1,000-fold with a blocking solution was added, followed by leaving to stand at room temperature for 50 minutes. The supernatant was removed, and after washing with 200 **µ**L of PBS-T three times, 100 **µ**L of 3,3',5,5'-tetramethylbenzidine (manufactured by Abcam plc.) was added, followed by leaving to stand at room temperature for 15 minutes. As a reaction stopping solution, 100 **µ**L of 0.5 M sulfuric acid was added, and the absorbance at 450 nm was measured with a multiplate reader (manufactured by Molecular Devices, LLC.). The results are shown in Figure 4.

### <Calculation of KD value by Bio-Layer Interferometry: BLI method>

The apparatus used was BLItz^{™} (manufactured by ForteBio). The tip of SA chip (manufactured by ForteBio) was kept in contact with purified water for 1 minute for hydration and was then kept in contact with a 1% BSA aqueous solution for 1 hour for blocking. The SA chip subjected to blocking treatment was set to the measuring unit of the BLItz^{™} main body, and measurement was performed according to the program shown in Table 14. The peptides of SEQ ID NOs: 1, 21, and 28 were used, and the concentration was adjusted to 100 **µ**M. The concentration of the GII.3 VLP was adjusted to 1 and 0.1 mg/mL. The measurement data were analyzed using the attached software, and the KD value was calculated based on the Ka and Kd values. The results are shown in Table 15.

**[Table 14]**

| Operation | Used solution | Time [sec] |
|---|---|---|
| Baseline | PBS | 30 |
| Peptide bonding | 100 **µ**M Peptide | 60 |
| Wash | PBS | 30 |
| Association | VLP | 90 |
| Dissociation | PBS | 60 |

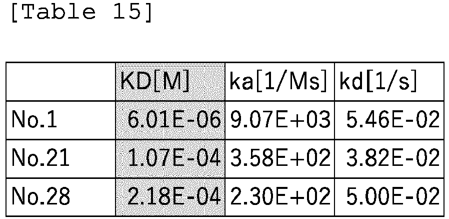

**[Table 16]**

| SEQ ID NO: | Sequence | Appearance frequency | SEQ ID NO: | Sequence | Appearance frequency | SEQ ID NO: | Sequence | Appearance frequency | SEQ ID NO: | Sequence | Appearance frequency | SEQ ID NO: | Sequence | Appearance frequency |
|---|---|---|---|---|---|---|---|---|---|---|---|---|---|---|
| 1 | GRRYYI | 23 | 55 | GTRHPS | 9 | 109 | GCHYQF | 7 | 163 | AFDGLG | 6 | 217 | NDHDHH | 6 |
| 2 | CLSNLA | 21 | 56 | HRRGPS | 9 | 110 | GFAISR | 7 | 164 | AKQRFT | 6 | 218 | NGHSHA | 6 |
| 3 | GHRLHS | 21 | 57 | HVRPFY | 9 | 111 | GGGVTT | 7 | 165 | ARNHGS | 6 | 219 | NKSFHR | 6 |
| 4 | HLRSIR | 18 | 58 | KFSHRK | 9 | 112 | GHFDAR | 7 | 166 | ATITVW | 6 | 220 | NPVHIK | 6 |
| 5 | RCSHLR | 18 | 59 | MAWIGS | 9 | 113 | GMSCGI | 7 | 167 | CLHFAL | 6 | 221 | NRVKPR | 6 |
| 6 | FLDTLG | 17 | 60 | NTHRHS | 9 | 114 | GSLDIM | 7 | 168 | DKVCKN | 6 | 222 | NSIAHR | 6 |
| 7 | GWHETE | 17 | 61 | PVRALC | 9 | 115 | GVNSGH | 7 | 169 | DRWGSK | 6 | 223 | NSRYFT | 6 |
| 8 | PCSYFS | 17 | 62 | RALAKR | 9 | 116 | HHRLHG | 7 | 170 | DSKLHN | 6 | 224 | PDRFCA | 6 |
| 9 | GEHAHS | 15 | 63 | REESFS | 9 | 117 | HHYRGL | 7 | 171 | DWPYSR | 6 | 225 | PWTVPN | 6 |
| 10 | GHHVSV | 15 | 64 | RKLFRN | 9 | 118 | HSSFSS | 7 | 172 | ECHHVH | 6 | 226 | QCELKD | 6 |
| 11 | GHPNPR | 15 | 65 | SGTMQE | 9 | 119 | HYRSKS | 7 | 173 | EEKTRR | 6 | 227 | QFSQLG | 6 |
| 12 | LNWSRS | 15 | 66 | SGYYRV | 9 | 120 | IGKGMV | 7 | 174 | FGFREL | 6 | 228 | QRVSFL | 6 |
| 13 | SVQSWK | 15 | 67 | SKHLAG | 9 | 121 | ILISSC | 7 | 175 | FPFYHR | 6 | 229 | QVIQRL | 6 |
| 14 | RSVRMH | 14 | 68 | SSPRSH | 9 | 122 | ISRRGH | 7 | 176 | FQYAHW | 6 | 230 | QYCFVG | 6 |
| 15 | SIGVDR | 14 | 69 | VSVSLP | 9 | 123 | KRGGMV | 7 | 177 | FTQDAV | 6 | 231 | RGGRTL | 6 |
| 16 | LSKHSR | 13 | 70 | AVGCHV | 8 | 124 | KVHGST | 7 | 178 | GAKELS | 6 | 232 | RGHPST | 6 |
| 17 | RIGHMR | 13 | 71 | CNLIAK | 8 | 125 | LASMWR | 7 | 179 | GCLSHH | 6 | 233 | RGTMWL | 6 |
| 18 | SRWCLS | 13 | 72 | DFGQSS | 8 | 126 | LFHPWV | 7 | 180 | GFGQWI | 6 | 234 | RHGQFF | 6 |
| 19 | CTYVVE | 12 | 73 | EWAVSK | 8 | 127 | LISRHD | 7 | 181 | GKVTFD | 6 | 235 | RHTQTL | 6 |
| 20 | DYVFCG | 12 | 74 | FPYARN | 8 | 128 | LWNMYD | 7 | 182 | GLLIPF | 6 | 236 | RIVLES | 6 |
| 21 | GKFFCH | 12 | 75 | FTMHTN | 8 | 129 | MHSSLS | 7 | 183 | GMDLRS | 6 | 237 | RKCIDR | 6 |
| 22 | GLRYRE | 12 | 76 | GGSHST | 8 | 130 | NASRFY | 7 | 184 | GPCNSA | 6 | 238 | RLHQCR | 6 |
| 23 | LKASIR | 12 | 77 | GMLRFP | 8 | 131 | NHCPSR | 7 | 185 | GRNAGR | 6 | 239 | RLRDPQ | 6 |
| 24 | LRSGVN | 12 | 78 | GQRLTV | 8 | 132 | NHDVRF | 7 | 186 | HDSRIR | 6 | 240 | RNHSRI | 6 |
| 25 | NLYDRF | 12 | 79 | GWYFHA | 8 | 133 | NKYSHR | 7 | 187 | HDYVSE | 6 | 241 | RSSAKR | 6 |
| 26 | TWDLTL | 12 | 80 | HICIRR | 8 | 134 | NPKAHL | 7 | 188 | HFRRRV | 6 | 242 | RTISGS | 6 |
| 27 | VHYRQT | 12 | 81 | HRSNGH | 8 | 135 | NRHLDP | 7 | 189 | HHNAPI | 6 | 243 | RTLLVQ | 6 |
| 28 | VMMQCP | 12 | 82 | ILGGHS | 8 | 136 | PVLHLH | 7 | 190 | HKASHW | 6 | 244 | RTVPSS | 6 |
| 29 | DLLNSK | 11 | 83 | KTRAIN | 8 | 137 | QDRVTS | 7 | 191 | HLAHKK | 6 | 245 | RYMPVA | 6 |
| 30 | DRYSAW | 11 | 84 | LASSGH | 8 | 138 | QLHRHK | 7 | 192 | HLGFHT | 6 | 246 | SAMFAS | 6 |
| 31 | HIPSRH | 11 | 85 | LHMKPI | 8 | 139 | RFLMRH | 7 | 193 | HMMSRL | 6 | 247 | SEHNRY | 6 |
| 32 | IMSSIG | 11 | 86 | MSTHKV | 8 | 140 | RFVKRR | 7 | 194 | HQTGRL | 6 | 248 | SHWRSY | 6 |
| 33 | SRHCVP | 11 | 87 | SIESCL | 8 | 141 | RHKVHH | 7 | 195 | HSSLVT | 6 | 249 | SIITRR | 6 |
| 34 | SVHTHR | 11 | 88 | SITVLY | 8 | 142 | RIVGSA | 7 | 196 | HVGLDK | 6 | 250 | SKVIRW | 6 |
| 35 | TKRQNL | 11 | 89 | SRSICS | 8 | 143 | RRCKLL | 7 | 197 | HWLRSV | 6 | 251 | SKYTAL | 6 |
| 36 | VNGVSH | 11 | 90 | SSCVMP | 8 | 144 | RRGDNY | 7 | 198 | HYRARY | 6 | 252 | SRSHWG | 6 |
| 37 | WEVALH | 11 | 91 | SSLALH | 8 | 145 | RRRTFH | 7 | 199 | IGRTFH | 6 | 253 | SSKSFA | 6 |
| 38 | AGYPRY | 10 | 92 | TGHIVW | 8 | 146 | RSAAGD | 7 | 200 | IHSPQK | 6 | 254 | STEFAR | 6 |
| 39 | EMSRHC | 10 | 93 | THACAH | 8 | 147 | RSSSGR | 7 | 201 | IKAHTR | 6 | 255 | STLVCP | 6 |
| 40 | FPFGST | 10 | 94 | TVASNN | 8 | 148 | RTGLLS | 7 | 202 | ILTHQH | 6 | 256 | STSVDW | 6 |
| 41 | GNIPGH | 10 | 95 | VGHVNW | 8 | 149 | SDRVIA | 7 | 203 | ITLIDT | 6 | 257 | TNRTVS | 6 |
| 42 | GNSPYT | 10 | 96 | VGRFPQ | 8 | 150 | STVRHR | 7 | 204 | KACAHL | 6 | 258 | TNYLSF | 6 |
| 43 | HIHGRE | 10 | 97 | VISLET | 8 | 151 | STYRSW | 7 | 205 | KHAWDL | 6 | 259 | VGMHHS | 6 |
| 44 | QSSKKF | 10 | 98 | VVWFTD | 8 | 152 | SWPFCT | 7 | 206 | KSMNNG | 6 | 260 | VMSPCN | 6 |
| 45 | RPFTML | 10 | 99 | VYIMNH | 8 | 153 | TTKYMW | 7 | 207 | LHHSSY | 6 | 261 | VPFPHH | 6 |
| 46 | RVSLYD | 10 | 100 | WDRPAF | 8 | 154 | TVLHHR | 7 | 208 | LLGKPV | 6 | 262 | VPLLSR | 6 |
| 47 | TTSHFK | 10 | 101 | YEGRND | 8 | 155 | VMVGPS | 7 | 209 | LNDMSF | 6 | 263 | VSDPVV | 6 |
| 48 | VDSTSV | 10 | 102 | AFTSHW | 7 | 156 | VRNVLH | 7 | 210 | LSLRSQ | 6 | 264 | WTMSPA | 6 |
| 49 | VETDGH | 10 | 103 | AHLHCD | 7 | 157 | VRPSNG | 7 | 211 | LTALSV | 6 | 265 | WVFVKH | 6 |
| 50 | VIEMLD | 10 | 104 | DRVQSR | 7 | 158 | VWYWTS | 7 | 212 | MDHEMF | 6 | 266 | YGKQRG | 6 |
| 51 | VMCPNR | 10 | 105 | EEMIVS | 7 | 159 | VYFIPD | 7 | 213 | MQFELV | 6 | 267 | YKASHG | 6 |
| 52 | VRYPEI | 10 | 106 | FPTGTT | 7 | 160 | WAGHRH | 7 | 214 | MSNKMV | 6 | 268 | YRSNHG | 6 |
| 53 | ACRSAF | 9 | 107 | FRECLY | 7 | 161 | WVYARR | 7 | 215 | NAVSLK | 6 | 269 | YYPDHP | 6 |
| 54 | FDHFYS | 9 | 108 | FWHARL | 7 | 162 | YRARMS | 7 | 216 | NCRTRP | 6 | | | |

## Claims

1. A norovirus-binding peptide consisting of an amino acid sequence selected from the group consisting of SEQ ID NOs: 1 to 269.

2. The norovirus-binding peptide according to Claim 1, wherein the amino acid sequence selected from the group consisting of SEQ ID NOs: 1 to 269 is an amino acid sequence selected from the group consisting of SEQ ID NOs: 1, 4, 14, 15, 17, 21 and 28.

3. A norovirus-binding peptide consisting of the amino acid sequence selected from the group consisting of SEQ ID NOs: 1 to 269 according to Claim 1 or 2 with a cysteine residue added to either or both of an N-terminus and a C-terminus thereof.

4. The norovirus-binding peptide according to Claim 3, wherein the cysteine residue is added to the N-terminus and the C-terminus of the amino acid sequence selected from the group consisting of SEQ ID NOs: 1 to 269.

5. The norovirus-binding peptide according to Claim 4, wherein the cysteine residue added to the N-terminus and the cysteine residue added to the C-terminus of the peptide consisting of an amino acid sequence selected from the group consisting of SEQ ID NOs: 1 to 269 are linked to each other via a disulfide bond to form a ring.

6. A norovirus-binding peptide consisting of an amino acid sequence of the peptide according to any one of Claims 1 to 5 with 1 to 20 amino acids added to either or both of the N-terminus and the C-terminus of the peptide.

7. A method for detecting norovirus comprising using the norovirus-binding peptide according to any one of Claims 1 to 6.

8. A norovirus detection kit comprising the norovirus-binding peptide according to any one of Claims 1 to 6.


SEQUENCE LISTING

<110>  KAO CORPORATION

<120>  NOROVIRUS-BINDING PEPTIDE

<130>  KS1661

<150>  2019-100756
<151>  2019-05-29

<160>  292

<170>  PatentIn version 3.5

<210>  1
<211>  6
<212>  PRT
<213>  Artificial Sequence

<220>
<223>  Designed peptide

<400>  1

Gly Arg Arg Tyr Tyr Ile
1               5

<210>  2
<211>  6
<212>  PRT
<213>  Artificial Sequence

<220>
<223>  Designed peptide

<400>  2

Cys Leu Ser Asn Leu Ala
1               5

<210>  3
<211>  6
<212>  PRT
<213>  Artificial Sequence

<220>
<223>  Designed peptide

<400>  3

Gly His Arg Leu His Ser
1               5

<210>  4
<211>  6
<212>  PRT
<213>  Artificial Sequence

<220>

<223>  Designed peptide

<400>  4

His Leu Arg Ser Ile Arg
1               5


<210>  5
<211>  6
<212>  PRT
<213>  Artificial Sequence

<220>
<223>  Designed peptide

<400>  5

Arg Cys Ser His Leu Arg
1               5


<210>  6
<211>  6
<212>  PRT
<213>  Artificial Sequence

<220>
<223>  Designed peptide

<400>  6

Phe Leu Asp Thr Leu Gly
1               5


<210>  7
<211>  6
<212>  PRT
<213>  Artificial Sequence

<220>
<223>  Designed peptide

<400>  7

Gly Trp His Glu Thr Glu
1               5


<210>  8
<211>  6
<212>  PRT
<213>  Artificial Sequence

<220>
<223>  Designed peptide

<400>  8

Pro Cys Ser Tyr Phe Ser
1               5

<210>  9
<211>  6
<212>  PRT
<213>  Artificial Sequence

<220>
<223>  Designed peptide

<400>  9

Gly Glu His Ala His Ser
1               5


<210>  10
<211>  6
<212>  PRT
<213>  Artificial Sequence

<220>
<223>  Designed peptide

<400>  10

Gly His His Val Ser Val
1               5


<210>  11
<211>  6
<212>  PRT
<213>  Artificial Sequence

<220>
<223>  Designed peptide

<400>  11

Gly His Pro Asn Pro Arg
1               5


<210>  12
<211>  6
<212>  PRT
<213>  Artificial Sequence

<220>
<223>  Designed peptide

<400>  12

Leu Asn Trp Ser Arg Ser
1               5


<210>  13
<211>  6
<212>  PRT
<213>  Artificial Sequence

<220>

<223>  Designed peptide

<400>  13

Ser Val Gln Ser Trp Lys
1               5


<210>  14
<211>  6
<212>  PRT
<213>  Artificial Sequence

<220>
<223>  Designed peptide

<400>  14

Arg Ser Val Arg Met His
1               5


<210>  15
<211>  6
<212>  PRT
<213>  Artificial Sequence

<220>
<223>  Designed peptide

<400>  15

Ser Ile Gly Val Asp Arg
1               5


<210>  16
<211>  6
<212>  PRT
<213>  Artificial Sequence

<220>
<223>  Designed peptide

<400>  16

Leu Ser Lys His Ser Arg
1               5


<210>  17
<211>  6
<212>  PRT
<213>  Artificial Sequence

<220>
<223>  Designed peptide

<400>  17

Arg Ile Gly His Met Arg
1               5

<210>  18
<211>  6
<212>  PRT
<213>  Artificial Sequence

<220>
<223>  Designed peptide

<400>  18

Ser Arg Trp Cys Leu Ser
1               5


<210>  19
<211>  6
<212>  PRT
<213>  Artificial Sequence

<220>
<223>  Designed peptide

<400>  19

Cys Thr Tyr Val Val Glu
1               5


<210>  20
<211>  6
<212>  PRT
<213>  Artificial Sequence

<220>
<223>  Designed peptide

<400>  20

Asp Tyr Val Phe Cys Gly
1               5


<210>  21
<211>  6
<212>  PRT
<213>  Artificial Sequence

<220>
<223>  Designed peptide

<400>  21

Gly Lys Phe Phe Cys His
1               5


<210>  22
<211>  6
<212>  PRT
<213>  Artificial Sequence

<220>

<223>  Designed peptide

<400>  22

Gly Leu Arg Tyr Arg Glu
1               5


<210>  23
<211>  6
<212>  PRT
<213>  Artificial Sequence

<220>
<223>  Designed peptide

<400>  23

Leu Lys Ala Ser Ile Arg
1               5


<210>  24
<211>  6
<212>  PRT
<213>  Artificial Sequence

<220>
<223>  Designed peptide

<400>  24

Leu Arg Ser Gly Val Asn
1               5


<210>  25
<211>  6
<212>  PRT
<213>  Artificial Sequence

<220>
<223>  Designed peptide

<400>  25

Asn Leu Tyr Asp Arg Phe
1               5


<210>  26
<211>  6
<212>  PRT
<213>  Artificial Sequence

<220>
<223>  Designed peptide

<400>  26

Thr Trp Asp Leu Thr Leu
1               5

<210>  27
<211>  6
<212>  PRT
<213>  Artificial Sequence

<220>
<223>  Designed peptide

<400>  27

Val His Tyr Arg Gln Thr
1               5


<210>  28
<211>  6
<212>  PRT
<213>  Artificial Sequence

<220>
<223>  Designed peptide

<400>  28

Val Met Met Gln Cys Pro
1               5


<210>  29
<211>  6
<212>  PRT
<213>  Artificial Sequence

<220>
<223>  Designed peptide

<400>  29

Asp Leu Leu Asn Ser Lys
1               5


<210>  30
<211>  6
<212>  PRT
<213>  Artificial Sequence

<220>
<223>  Designed peptide

<400>  30

Asp Arg Tyr Ser Ala Trp
1               5


<210>  31
<211>  6
<212>  PRT
<213>  Artificial Sequence

<220>

<223>  Designed peptide

<400>  31

His Ile Pro Ser Arg His
1               5


<210>  32
<211>  6
<212>  PRT
<213>  Artificial Sequence

<220>
<223>  Designed peptide

<400>  32

Ile Met Ser Ser Ile Gly
1               5


<210>  33
<211>  6
<212>  PRT
<213>  Artificial Sequence

<220>
<223>  Designed peptide

<400>  33

Ser Arg His Cys Val Pro
1               5


<210>  34
<211>  6
<212>  PRT
<213>  Artificial Sequence

<220>
<223>  Designed peptide

<400>  34

Ser Val His Thr His Arg
1               5


<210>  35
<211>  6
<212>  PRT
<213>  Artificial Sequence

<220>
<223>  Designed peptide

<400>  35

Thr Lys Arg Gln Asn Leu
1               5

<210>  36
<211>  6
<212>  PRT
<213>  Artificial Sequence

<220>
<223>  Designed peptide

<400>  36

Val Asn Gly Val Ser His
1               5


<210>  37
<211>  6
<212>  PRT
<213>  Artificial Sequence

<220>
<223>  Designed peptide

<400>  37

Trp Glu Val Ala Leu His
1               5


<210>  38
<211>  6
<212>  PRT
<213>  Artificial Sequence

<220>
<223>  Designed peptide

<400>  38

Ala Gly Tyr Pro Arg Tyr
1               5


<210>  39
<211>  6
<212>  PRT
<213>  Artificial Sequence

<220>
<223>  Designed peptide

<400>  39

Glu Met Ser Arg His Cys
1               5


<210>  40
<211>  6
<212>  PRT
<213>  Artificial Sequence

<220>

<223>  Designed peptide

<400>  40

Phe Pro Phe Gly Ser Thr
1               5


<210>  41
<211>  6
<212>  PRT
<213>  Artificial Sequence

<220>
<223>  Designed peptide

<400>  41

Gly Asn Ile Pro Gly His
1               5


<210>  42
<211>  6
<212>  PRT
<213>  Artificial Sequence

<220>
<223>  Designed peptide

<400>  42

Gly Asn Ser Pro Tyr Thr
1               5


<210>  43
<211>  6
<212>  PRT
<213>  Artificial Sequence

<220>
<223>  Designed peptide

<400>  43

His Ile His Gly Arg Glu
1               5


<210>  44
<211>  6
<212>  PRT
<213>  Artificial Sequence

<220>
<223>  Designed peptide

<400>  44

Gln Ser Ser Lys Lys Phe
1               5

<210>  45
<211>  6
<212>  PRT
<213>  Artificial Sequence

<220>
<223>  Designed peptide

<400>  45

Arg Pro Phe Thr Met Leu
1               5


<210>  46
<211>  6
<212>  PRT
<213>  Artificial Sequence

<220>
<223>  Designed peptide

<400>  46

Arg Val Ser Leu Tyr Asp
1               5


<210>  47
<211>  6
<212>  PRT
<213>  Artificial Sequence

<220>
<223>  Designed peptide

<400>  47

Thr Thr Ser His Phe Lys
1               5


<210>  48
<211>  6
<212>  PRT
<213>  Artificial Sequence

<220>
<223>  Designed peptide

<400>  48

Val Asp Ser Thr Ser Val
1               5


<210>  49
<211>  6
<212>  PRT
<213>  Artificial Sequence

<220>

<223>  Designed peptide

<400>  49

Val Glu Thr Asp Gly His
1               5


<210>  50
<211>  6
<212>  PRT
<213>  Artificial Sequence

<220>
<223>  Designed peptide

<400>  50

Val Ile Glu Met Leu Asp
1               5


<210>  51
<211>  6
<212>  PRT
<213>  Artificial Sequence

<220>
<223>  Designed peptide

<400>  51

Val Met Cys Pro Asn Arg
1               5


<210>  52
<211>  6
<212>  PRT
<213>  Artificial Sequence

<220>
<223>  Designed peptide

<400>  52

Val Arg Tyr Pro Glu Ile
1               5


<210>  53
<211>  6
<212>  PRT
<213>  Artificial Sequence

<220>
<223>  Designed peptide

<400>  53

Ala Cys Arg Ser Ala Phe
1               5

<210>  54
<211>  6
<212>  PRT
<213>  Artificial Sequence

<220>
<223>  Designed peptide

<400>  54

Phe Asp His Phe Tyr Ser
1               5


<210>  55
<211>  6
<212>  PRT
<213>  Artificial Sequence

<220>
<223>  Designed peptide

<400>  55

Gly Thr Arg His Pro Ser
1               5


<210>  56
<211>  6
<212>  PRT
<213>  Artificial Sequence

<220>
<223>  Designed peptide

<400>  56

His Arg Arg Gly Pro Ser
1               5


<210>  57
<211>  6
<212>  PRT
<213>  Artificial Sequence

<220>
<223>  Designed peptide

<400>  57

His Val Arg Pro Phe Tyr
1               5


<210>  58
<211>  6
<212>  PRT
<213>  Artificial Sequence

<220>

<223>  Designed peptide

<400>  58

Lys Phe Ser His Arg Lys
1               5


<210>  59
<211>  6
<212>  PRT
<213>  Artificial Sequence

<220>
<223>  Designed peptide

<400>  59

Met Ala Trp Ile Gly Ser
1               5


<210>  60
<211>  6
<212>  PRT
<213>  Artificial Sequence

<220>
<223>  Designed peptide

<400>  60

Asn Thr His Arg His Ser
1               5


<210>  61
<211>  6
<212>  PRT
<213>  Artificial Sequence

<220>
<223>  Designed peptide

<400>  61

Pro Val Arg Ala Leu Cys
1               5


<210>  62
<211>  6
<212>  PRT
<213>  Artificial Sequence

<220>
<223>  Designed peptide

<400>  62

Arg Ala Leu Ala Lys Arg
1               5

<210>  63
<211>  6
<212>  PRT
<213>  Artificial Sequence

<220>
<223>  Designed peptide

<400>  63

Arg Glu Glu Ser Phe Ser
1               5


<210>  64
<211>  6
<212>  PRT
<213>  Artificial Sequence

<220>
<223>  Designed peptide

<400>  64

Arg Lys Leu Phe Arg Asn
1               5


<210>  65
<211>  6
<212>  PRT
<213>  Artificial Sequence

<220>
<223>  Designed peptide

<400>  65

Ser Gly Thr Met Gln Glu
1               5


<210>  66
<211>  6
<212>  PRT
<213>  Artificial Sequence

<220>
<223>  Designed peptide

<400>  66

Ser Gly Tyr Tyr Arg Val
1               5


<210>  67
<211>  6
<212>  PRT
<213>  Artificial Sequence

<220>

<223>  Designed peptide

<400>  67

Ser Lys His Leu Ala Gly
1               5

<210>  68
<211>  6
<212>  PRT
<213>  Artificial Sequence

<220>
<223>  Designed peptide

<400>  68

Ser Ser Pro Arg Ser His
1               5

<210>  69
<211>  6
<212>  PRT
<213>  Artificial Sequence

<220>
<223>  Designed peptide

<400>  69

Val Ser Val Ser Leu Pro
1               5

<210>  70
<211>  6
<212>  PRT
<213>  Artificial Sequence

<220>
<223>  Designed peptide

<400>  70

Ala Val Gly Cys His Val
1               5

<210>  71
<211>  6
<212>  PRT
<213>  Artificial Sequence

<220>
<223>  Designed peptide

<400>  71

Cys Asn Leu Ile Ala Lys
1               5

<210>  72
<211>  6
<212>  PRT
<213>  Artificial Sequence

<220>
<223>  Designed peptide

<400>  72

Asp Phe Gly Gln Ser Ser
1               5


<210>  73
<211>  6
<212>  PRT
<213>  Artificial Sequence

<220>
<223>  Designed peptide

<400>  73

Glu Trp Ala Val Ser Lys
1               5


<210>  74
<211>  6
<212>  PRT
<213>  Artificial Sequence

<220>
<223>  Designed peptide

<400>  74

Phe Pro Tyr Ala Arg Asn
1               5


<210>  75
<211>  6
<212>  PRT
<213>  Artificial Sequence

<220>
<223>  Designed peptide

<400>  75

Phe Thr Met His Thr Asn
1               5


<210>  76
<211>  6
<212>  PRT
<213>  Artificial Sequence

<220>

<223>  Designed peptide

<400>  76

Gly Gly Ser His Ser Thr
1               5

<210>  77
<211>  6
<212>  PRT
<213>  Artificial Sequence

<220>
<223>  Designed peptide

<400>  77

Gly Met Leu Arg Phe Pro
1               5

<210>  78
<211>  6
<212>  PRT
<213>  Artificial Sequence

<220>
<223>  Designed peptide

<400>  78

Gly Gln Arg Leu Thr Val
1               5

<210>  79
<211>  6
<212>  PRT
<213>  Artificial Sequence

<220>
<223>  Designed peptide

<400>  79

Gly Trp Tyr Phe His Ala
1               5

<210>  80
<211>  6
<212>  PRT
<213>  Artificial Sequence

<220>
<223>  Designed peptide

<400>  80

His Ile Cys Ile Arg Arg
1               5

<210>  81
<211>  6
<212>  PRT
<213>  Artificial Sequence

<220>
<223>  Designed peptide

<400>  81

His Arg Ser Asn Gly His
1               5


<210>  82
<211>  6
<212>  PRT
<213>  Artificial Sequence

<220>
<223>  Designed peptide

<400>  82

Ile Leu Gly Gly His Ser
1               5


<210>  83
<211>  6
<212>  PRT
<213>  Artificial Sequence

<220>
<223>  Designed peptide

<400>  83

Lys Thr Arg Ala Ile Asn
1               5


<210>  84
<211>  6
<212>  PRT
<213>  Artificial Sequence

<220>
<223>  Designed peptide

<400>  84

Leu Ala Ser Ser Gly His
1               5


<210>  85
<211>  6
<212>  PRT
<213>  Artificial Sequence

<220>

<223>  Designed peptide

<400>  85

Leu His Met Lys Pro Ile
1               5


<210>  86
<211>  6
<212>  PRT
<213>  Artificial Sequence

<220>
<223>  Designed peptide

<400>  86

Met Ser Thr His Lys Val
1               5


<210>  87
<211>  6
<212>  PRT
<213>  Artificial Sequence

<220>
<223>  Designed peptide

<400>  87

Ser Ile Glu Ser Cys Leu
1               5


<210>  88
<211>  6
<212>  PRT
<213>  Artificial Sequence

<220>
<223>  Designed peptide

<400>  88

Ser Ile Thr Val Leu Tyr
1               5


<210>  89
<211>  6
<212>  PRT
<213>  Artificial Sequence

<220>
<223>  Designed peptide

<400>  89

Ser Arg Ser Ile Cys Ser
1               5

<210>  90
<211>  6
<212>  PRT
<213>  Artificial Sequence

<220>
<223>  Designed peptide

<400>  90

Ser Ser Cys Val Met Pro
1               5


<210>  91
<211>  6
<212>  PRT
<213>  Artificial Sequence

<220>
<223>  Designed peptide

<400>  91

Ser Ser Leu Ala Leu His
1               5


<210>  92
<211>  6
<212>  PRT
<213>  Artificial Sequence

<220>
<223>  Designed peptide

<400>  92

Thr Gly His Ile Val Trp
1               5


<210>  93
<211>  6
<212>  PRT
<213>  Artificial Sequence

<220>
<223>  Designed peptide

<400>  93

Thr His Ala Cys Ala His
1               5


<210>  94
<211>  6
<212>  PRT
<213>  Artificial Sequence

<220>

<223>  Designed peptide

<400>  94

Thr Val Ala Ser Asn Asn
1               5


<210>  95
<211>  6
<212>  PRT
<213>  Artificial Sequence

<220>
<223>  Designed peptide

<400>  95

Val Gly His Val Asn Trp
1               5


<210>  96
<211>  6
<212>  PRT
<213>  Artificial Sequence

<220>
<223>  Designed peptide

<400>  96

Val Gly Arg Phe Pro Gln
1               5


<210>  97
<211>  6
<212>  PRT
<213>  Artificial Sequence

<220>
<223>  Designed peptide

<400>  97

Val Ile Ser Leu Glu Thr
1               5


<210>  98
<211>  6
<212>  PRT
<213>  Artificial Sequence

<220>
<223>  Designed peptide

<400>  98

Val Val Trp Phe Thr Asp
1               5

<210>  99
<211>  6
<212>  PRT
<213>  Artificial Sequence

<220>
<223>  Designed peptide

<400>  99

Val Tyr Ile Met Asn His
1               5


<210>  100
<211>  6
<212>  PRT
<213>  Artificial Sequence

<220>
<223>  Designed peptide

<400>  100

Trp Asp Arg Pro Ala Phe
1               5


<210>  101
<211>  6
<212>  PRT
<213>  Artificial Sequence

<220>
<223>  Designed peptide

<400>  101

Tyr Glu Gly Arg Asn Asp
1               5


<210>  102
<211>  6
<212>  PRT
<213>  Artificial Sequence

<220>
<223>  Designed peptide

<400>  102

Ala Phe Thr Ser His Trp
1               5


<210>  103
<211>  6
<212>  PRT
<213>  Artificial Sequence

<220>

<223>  Designed peptide

<400>  103

Ala His Leu His Cys Asp
1               5


<210>  104
<211>  6
<212>  PRT
<213>  Artificial Sequence

<220>
<223>  Designed peptide

<400>  104

Asp Arg Val Gln Ser Arg
1               5


<210>  105
<211>  6
<212>  PRT
<213>  Artificial Sequence

<220>
<223>  Designed peptide

<400>  105

Glu Glu Met Ile Val Ser
1               5


<210>  106
<211>  6
<212>  PRT
<213>  Artificial Sequence

<220>
<223>  Designed peptide

<400>  106

Phe Pro Thr Gly Thr Thr
1               5


<210>  107
<211>  6
<212>  PRT
<213>  Artificial Sequence

<220>
<223>  Designed peptide

<400>  107

Phe Arg Glu Cys Leu Tyr
1               5

<210>  108
<211>  6
<212>  PRT
<213>  Artificial Sequence

<220>
<223>  Designed peptide

<400>  108

Phe Trp His Ala Arg Leu
1               5


<210>  109
<211>  6
<212>  PRT
<213>  Artificial Sequence

<220>
<223>  Designed peptide

<400>  109

Gly Cys His Tyr Gln Phe
1               5


<210>  110
<211>  6
<212>  PRT
<213>  Artificial Sequence

<220>
<223>  Designed peptide

<400>  110

Gly Phe Ala Ile Ser Arg
1               5


<210>  111
<211>  6
<212>  PRT
<213>  Artificial Sequence

<220>
<223>  Designed peptide

<400>  111

Gly Gly Gly Val Thr Thr
1               5


<210>  112
<211>  6
<212>  PRT
<213>  Artificial Sequence

<220>

<223>  Designed peptide

<400>  112

Gly His Phe Asp Ala Arg
1               5


<210>  113
<211>  6
<212>  PRT
<213>  Artificial Sequence

<220>
<223>  Designed peptide

<400>  113

Gly Met Ser Cys Gly Ile
1               5


<210>  114
<211>  6
<212>  PRT
<213>  Artificial Sequence

<220>
<223>  Designed peptide

<400>  114

Gly Ser Leu Asp Ile Met
1               5


<210>  115
<211>  6
<212>  PRT
<213>  Artificial Sequence

<220>
<223>  Designed peptide

<400>  115

Gly Val Asn Ser Gly His
1               5


<210>  116
<211>  6
<212>  PRT
<213>  Artificial Sequence

<220>
<223>  Designed peptide

<400>  116

His His Arg Leu His Gly
1               5

<210>  117
<211>  6
<212>  PRT
<213>  Artificial Sequence

<220>
<223>  Designed peptide

<400>  117

His His Tyr Arg Gly Leu
1               5


<210>  118
<211>  6
<212>  PRT
<213>  Artificial Sequence

<220>
<223>  Designed peptide

<400>  118

His Ser Ser Phe Ser Ser
1               5


<210>  119
<211>  6
<212>  PRT
<213>  Artificial Sequence

<220>
<223>  Designed peptide

<400>  119

His Tyr Arg Ser Lys Ser
1               5


<210>  120
<211>  6
<212>  PRT
<213>  Artificial Sequence

<220>
<223>  Designed peptide

<400>  120

Ile Gly Lys Gly Met Val
1               5


<210>  121
<211>  6
<212>  PRT
<213>  Artificial Sequence

<220>

<223>  Designed peptide

<400>  121

Ile Leu Ile Ser Ser Cys
1               5


<210>  122
<211>  6
<212>  PRT
<213>  Artificial Sequence

<220>
<223>  Designed peptide

<400>  122

Ile Ser Arg Arg Gly His
1               5


<210>  123
<211>  6
<212>  PRT
<213>  Artificial Sequence

<220>
<223>  Designed peptide

<400>  123

Lys Arg Gly Gly Met Val
1               5


<210>  124
<211>  6
<212>  PRT
<213>  Artificial Sequence

<220>
<223>  Designed peptide

<400>  124

Lys Val His Gly Ser Thr
1               5


<210>  125
<211>  6
<212>  PRT
<213>  Artificial Sequence

<220>
<223>  Designed peptide

<400>  125

Leu Ala Ser Met Trp Arg
1               5

<210>  126
<211>  6
<212>  PRT
<213>  Artificial Sequence

<220>
<223>  Designed peptide

<400>  126

Leu Phe His Pro Trp Val
1               5


<210>  127
<211>  6
<212>  PRT
<213>  Artificial Sequence

<220>
<223>  Designed peptide

<400>  127

Leu Ile Ser Arg His Asp
1               5


<210>  128
<211>  6
<212>  PRT
<213>  Artificial Sequence

<220>
<223>  Designed peptide

<400>  128

Leu Trp Asn Met Tyr Asp
1               5


<210>  129
<211>  6
<212>  PRT
<213>  Artificial Sequence

<220>
<223>  Designed peptide

<400>  129

Met His Ser Ser Leu Ser
1               5


<210>  130
<211>  6
<212>  PRT
<213>  Artificial Sequence

<220>

<223>  Designed peptide

<400>  130

Asn Ala Ser Arg Phe Tyr
1               5


<210>  131
<211>  6
<212>  PRT
<213>  Artificial Sequence

<220>
<223>  Designed peptide

<400>  131

Asn His Cys Pro Ser Arg
1               5


<210>  132
<211>  6
<212>  PRT
<213>  Artificial Sequence

<220>
<223>  Designed peptide

<400>  132

Asn His Asp Val Arg Phe
1               5


<210>  133
<211>  6
<212>  PRT
<213>  Artificial Sequence

<220>
<223>  Designed peptide

<400>  133

Asn Lys Tyr Ser His Arg
1               5


<210>  134
<211>  6
<212>  PRT
<213>  Artificial Sequence

<220>
<223>  Designed peptide

<400>  134

Asn Pro Lys Ala His Leu
1               5

<210>  135
<211>  6
<212>  PRT
<213>  Artificial Sequence

<220>
<223>  Designed peptide

<400>  135

Asn Arg His Leu Asp Pro
1               5


<210>  136
<211>  6
<212>  PRT
<213>  Artificial Sequence

<220>
<223>  Designed peptide

<400>  136

Pro Val Leu His Leu His
1               5


<210>  137
<211>  6
<212>  PRT
<213>  Artificial Sequence

<220>
<223>  Designed peptide

<400>  137

Gln Asp Arg Val Thr Ser
1               5


<210>  138
<211>  6
<212>  PRT
<213>  Artificial Sequence

<220>
<223>  Designed peptide

<400>  138

Gln Leu His Arg His Lys
1               5


<210>  139
<211>  6
<212>  PRT
<213>  Artificial Sequence

<220>

<223>  Designed peptide

<400>  139

Arg Phe Leu Met Arg His
1               5


<210>  140
<211>  6
<212>  PRT
<213>  Artificial Sequence

<220>
<223>  Designed peptide

<400>  140

Arg Phe Val Lys Arg Arg
1               5


<210>  141
<211>  6
<212>  PRT
<213>  Artificial Sequence

<220>
<223>  Designed peptide

<400>  141

Arg His Lys Val His His
1               5


<210>  142
<211>  6
<212>  PRT
<213>  Artificial Sequence

<220>
<223>  Designed peptide

<400>  142

Arg Ile Val Gly Ser Ala
1               5


<210>  143
<211>  6
<212>  PRT
<213>  Artificial Sequence

<220>
<223>  Designed peptide

<400>  143

Arg Arg Cys Lys Leu Leu
1               5

<210>  144
<211>  6
<212>  PRT
<213>  Artificial Sequence

<220>
<223>  Designed peptide

<400>  144

Arg Arg Gly Asp Asn Tyr
1               5


<210>  145
<211>  6
<212>  PRT
<213>  Artificial Sequence

<220>
<223>  Designed peptide

<400>  145

Arg Arg Arg Thr Phe His
1               5


<210>  146
<211>  6
<212>  PRT
<213>  Artificial Sequence

<220>
<223>  Designed peptide

<400>  146

Arg Ser Ala Ala Gly Asp
1               5


<210>  147
<211>  6
<212>  PRT
<213>  Artificial Sequence

<220>
<223>  Designed peptide

<400>  147

Arg Ser Ser Ser Gly Arg
1               5


<210>  148
<211>  6
<212>  PRT
<213>  Artificial Sequence

<220>

<223>  Designed peptide

<400>  148

Arg Thr Gly Leu Leu Ser
1               5


<210>  149
<211>  6
<212>  PRT
<213>  Artificial Sequence

<220>
<223>  Designed peptide

<400>  149

Ser Asp Arg Val Ile Ala
1               5


<210>  150
<211>  6
<212>  PRT
<213>  Artificial Sequence

<220>
<223>  Designed peptide

<400>  150

Ser Thr Val Arg His Arg
1               5


<210>  151
<211>  6
<212>  PRT
<213>  Artificial Sequence

<220>
<223>  Designed peptide

<400>  151

Ser Thr Tyr Arg Ser Trp
1               5


<210>  152
<211>  6
<212>  PRT
<213>  Artificial Sequence

<220>
<223>  Designed peptide

<400>  152

Ser Trp Pro Phe Cys Thr
1               5

<210>  153
<211>  6
<212>  PRT
<213>  Artificial Sequence

<220>
<223>  Designed peptide

<400>  153

Thr Thr Lys Tyr Met Trp
1               5


<210>  154
<211>  6
<212>  PRT
<213>  Artificial Sequence

<220>
<223>  Designed peptide

<400>  154

Thr Val Leu His His Arg
1               5


<210>  155
<211>  6
<212>  PRT
<213>  Artificial Sequence

<220>
<223>  Designed peptide

<400>  155

Val Met Val Gly Pro Ser
1               5


<210>  156
<211>  6
<212>  PRT
<213>  Artificial Sequence

<220>
<223>  Designed peptide

<400>  156

Val Arg Asn Leu Val His
1               5


<210>  157
<211>  6
<212>  PRT
<213>  Artificial Sequence

<220>

<223>  Designed peptide

<400>  157

Val Arg Pro Ser Asn Gly
1               5


<210>  158
<211>  6
<212>  PRT
<213>  Artificial Sequence

<220>
<223>  Designed peptide

<400>  158

Val Trp Tyr Trp Thr Ser
1               5


<210>  159
<211>  6
<212>  PRT
<213>  Artificial Sequence

<220>
<223>  Designed peptide

<400>  159

Val Tyr Phe Ile Pro Asp
1               5


<210>  160
<211>  6
<212>  PRT
<213>  Artificial Sequence

<220>
<223>  Designed peptide

<400>  160

Trp Ala Gly His Arg His
1               5


<210>  161
<211>  6
<212>  PRT
<213>  Artificial Sequence

<220>
<223>  Designed peptide

<400>  161

Trp Val Tyr Ala Arg Arg
1               5

<210>  162
<211>  6
<212>  PRT
<213>  Artificial Sequence

<220>
<223>  Designed peptide

<400>  162

Tyr Arg Ala Arg Met Ser
1               5


<210>  163
<211>  6
<212>  PRT
<213>  Artificial Sequence

<220>
<223>  Designed peptide

<400>  163

Ala Phe Asp Gly Leu Gly
1               5


<210>  164
<211>  6
<212>  PRT
<213>  Artificial Sequence

<220>
<223>  Designed peptide

<400>  164

Ala Lys Gln Arg Phe Thr
1               5


<210>  165
<211>  6
<212>  PRT
<213>  Artificial Sequence

<220>
<223>  Designed peptide

<400>  165

Ala Arg Asn His Gly Ser
1               5


<210>  166
<211>  6
<212>  PRT
<213>  Artificial Sequence

<220>

<223>  Designed peptide

<400>  166

Ala Thr Ile Thr Val Trp
1               5


<210>  167
<211>  6
<212>  PRT
<213>  Artificial Sequence

<220>
<223>  Designed peptide

<400>  167

Cys Leu His Phe Ala Leu
1               5


<210>  168
<211>  6
<212>  PRT
<213>  Artificial Sequence

<220>
<223>  Designed peptide

<400>  168

Asp Lys Val Cys Lys Asn
1               5


<210>  169
<211>  6
<212>  PRT
<213>  Artificial Sequence

<220>
<223>  Designed peptide

<400>  169

Asp Arg Trp Gly Ser Lys
1               5


<210>  170
<211>  6
<212>  PRT
<213>  Artificial Sequence

<220>
<223>  Designed peptide

<400>  170

Asp Ser Lys Leu His Asn
1               5

<210>  171
<211>  6
<212>  PRT
<213>  Artificial Sequence

<220>
<223>  Designed peptide

<400>  171

Asp Trp Pro Tyr Ser Arg
1               5


<210>  172
<211>  6
<212>  PRT
<213>  Artificial Sequence

<220>
<223>  Designed peptide

<400>  172

Glu Cys His His Val His
1               5


<210>  173
<211>  6
<212>  PRT
<213>  Artificial Sequence

<220>
<223>  Designed peptide

<400>  173

Glu Glu Lys Thr Arg Arg
1               5


<210>  174
<211>  6
<212>  PRT
<213>  Artificial Sequence

<220>
<223>  Designed peptide

<400>  174

Phe Gly Phe Arg Glu Leu
1               5


<210>  175
<211>  6
<212>  PRT
<213>  Artificial Sequence

<220>

<223>  Designed peptide

<400>  175

Phe Pro Phe Tyr His Arg
1               5


<210>  176
<211>  6
<212>  PRT
<213>  Artificial Sequence

<220>
<223>  Designed peptide

<400>  176

Phe Gln Tyr Ala His Trp
1               5


<210>  177
<211>  6
<212>  PRT
<213>  Artificial Sequence

<220>
<223>  Designed peptide

<400>  177

Phe Thr Gln Asp Ala Val
1               5


<210>  178
<211>  6
<212>  PRT
<213>  Artificial Sequence

<220>
<223>  Designed peptide

<400>  178

Gly Ala Lys Glu Leu Ser
1               5


<210>  179
<211>  6
<212>  PRT
<213>  Artificial Sequence

<220>
<223>  Designed peptide

<400>  179

Gly Cys Leu Ser His His
1               5

<210>  180
<211>  6
<212>  PRT
<213>  Artificial Sequence

<220>
<223>  Designed peptide

<400>  180

Gly Phe Gly Gln Trp Ile
1               5


<210>  181
<211>  6
<212>  PRT
<213>  Artificial Sequence

<220>
<223>  Designed peptide

<400>  181

Gly Lys Val Thr Phe Asp
1               5


<210>  182
<211>  6
<212>  PRT
<213>  Artificial Sequence

<220>
<223>  Designed peptide

<400>  182

Gly Leu Leu Ile Pro Phe
1               5


<210>  183
<211>  6
<212>  PRT
<213>  Artificial Sequence

<220>
<223>  Designed peptide

<400>  183

Gly Met Asp Leu Arg Ser
1               5


<210>  184
<211>  6
<212>  PRT
<213>  Artificial Sequence

<220>

<223>  Designed peptide

<400>  184

Gly Pro Cys Asn Ser Ala
1               5

<210>  185
<211>  6
<212>  PRT
<213>  Artificial Sequence

<220>
<223>  Designed peptide

<400>  185

Gly Arg Asn Ala Gly Arg
1               5

<210>  186
<211>  6
<212>  PRT
<213>  Artificial Sequence

<220>
<223>  Designed peptide

<400>  186

His Asp Ser Arg Ile Arg
1               5

<210>  187
<211>  6
<212>  PRT
<213>  Artificial Sequence

<220>
<223>  Designed peptide

<400>  187

His Asp Tyr Val Ser Glu
1               5

<210>  188
<211>  6
<212>  PRT
<213>  Artificial Sequence

<220>
<223>  Designed peptide

<400>  188

His Phe Arg Arg Arg Val
1               5

<210>  189
<211>  6
<212>  PRT
<213>  Artificial Sequence

<220>
<223>  Designed peptide

<400>  189

His His Asn Ala Pro Ile
1               5


<210>  190
<211>  6
<212>  PRT
<213>  Artificial Sequence

<220>
<223>  Designed peptide

<400>  190

His Lys Ala Ser His Trp
1               5


<210>  191
<211>  6
<212>  PRT
<213>  Artificial Sequence

<220>
<223>  Designed peptide

<400>  191

His Leu Ala His Lys Lys
1               5


<210>  192
<211>  6
<212>  PRT
<213>  Artificial Sequence

<220>
<223>  Designed peptide

<400>  192

His Leu Gly Phe His Thr
1               5


<210>  193
<211>  6
<212>  PRT
<213>  Artificial Sequence

<220>

<223>  Designed peptide

<400>  193

His Met Met Ser Arg Leu
1               5


<210>  194
<211>  6
<212>  PRT
<213>  Artificial Sequence

<220>
<223>  Designed peptide

<400>  194

His Gln Thr Gly Arg Leu
1               5


<210>  195
<211>  6
<212>  PRT
<213>  Artificial Sequence

<220>
<223>  Designed peptide

<400>  195

His Ser Ser Leu Val Thr
1               5


<210>  196
<211>  6
<212>  PRT
<213>  Artificial Sequence

<220>
<223>  Designed peptide

<400>  196

His Val Gly Leu Asp Lys
1               5


<210>  197
<211>  6
<212>  PRT
<213>  Artificial Sequence

<220>
<223>  Designed peptide

<400>  197

His Trp Leu Arg Ser Val
1               5

<210>  198
<211>  6
<212>  PRT
<213>  Artificial Sequence

<220>
<223>  Designed peptide

<400>  198

His Tyr Arg Ala Arg Tyr
1               5


<210>  199
<211>  6
<212>  PRT
<213>  Artificial Sequence

<220>
<223>  Designed peptide

<400>  199

Ile Gly Arg Thr Phe His
1               5


<210>  200
<211>  6
<212>  PRT
<213>  Artificial Sequence

<220>
<223>  Designed peptide

<400>  200

Ile His Ser Pro Gln Lys
1               5


<210>  201
<211>  6
<212>  PRT
<213>  Artificial Sequence

<220>
<223>  Designed peptide

<400>  201

Ile Lys Ala His Thr Arg
1               5


<210>  202
<211>  6
<212>  PRT
<213>  Artificial Sequence

<220>

<223>  Designed peptide

<400>  202

Ile Leu Thr His Gln His
1               5


<210>  203
<211>  6
<212>  PRT
<213>  Artificial Sequence

<220>
<223>  Designed peptide

<400>  203

Ile Thr Leu Ile Asp Thr
1               5


<210>  204
<211>  6
<212>  PRT
<213>  Artificial Sequence

<220>
<223>  Designed peptide

<400>  204

Lys Ala Cys Ala His Leu
1               5


<210>  205
<211>  6
<212>  PRT
<213>  Artificial Sequence

<220>
<223>  Designed peptide

<400>  205

Lys His Ala Trp Asp Leu
1               5


<210>  206
<211>  6
<212>  PRT
<213>  Artificial Sequence

<220>
<223>  Designed peptide

<400>  206

Lys Ser Met Asn Asn Gly
1               5

<210>  207
<211>  6
<212>  PRT
<213>  Artificial Sequence

<220>
<223>  Designed peptide

<400>  207

Leu His His Ser Ser Tyr
1               5


<210>  208
<211>  6
<212>  PRT
<213>  Artificial Sequence

<220>
<223>  Designed peptide

<400>  208

Leu Leu Gly Lys Pro Val
1               5


<210>  209
<211>  6
<212>  PRT
<213>  Artificial Sequence

<220>
<223>  Designed peptide

<400>  209

Leu Asn Asp Met Ser Phe
1               5


<210>  210
<211>  6
<212>  PRT
<213>  Artificial Sequence

<220>
<223>  Designed peptide

<400>  210

Leu Ser Leu Arg Ser Gln
1               5


<210>  211
<211>  6
<212>  PRT
<213>  Artificial Sequence

<220>

<223>  Designed peptide

<400>  211

Leu Thr Ala Leu Ser Val
1               5


<210>  212
<211>  6
<212>  PRT
<213>  Artificial Sequence

<220>
<223>  Designed peptide

<400>  212

Met Asp His Glu Met Phe
1               5


<210>  213
<211>  6
<212>  PRT
<213>  Artificial Sequence

<220>
<223>  Designed peptide

<400>  213

Met Gln Phe Glu Leu Val
1               5


<210>  214
<211>  6
<212>  PRT
<213>  Artificial Sequence

<220>
<223>  Designed peptide

<400>  214

Met Ser Asn Lys Met Val
1               5


<210>  215
<211>  6
<212>  PRT
<213>  Artificial Sequence

<220>
<223>  Designed peptide

<400>  215

Asn Ala Val Ser Leu Lys
1               5

<210>  216
<211>  6
<212>  PRT
<213>  Artificial Sequence

<220>
<223>  Designed peptide

<400>  216

Asn Cys Arg Thr Arg Pro
1               5


<210>  217
<211>  6
<212>  PRT
<213>  Artificial Sequence

<220>
<223>  Designed peptide

<400>  217

Asn Asp His Asp His His
1               5


<210>  218
<211>  6
<212>  PRT
<213>  Artificial Sequence

<220>
<223>  Designed peptide

<400>  218

Asn Gly His Ser His Ala
1               5


<210>  219
<211>  6
<212>  PRT
<213>  Artificial Sequence

<220>
<223>  Designed peptide

<400>  219

Asn Lys Ser Phe His Arg
1               5


<210>  220
<211>  6
<212>  PRT
<213>  Artificial Sequence

<220>

<223>  Designed peptide

<400>  220

Asn Pro Val His Ile Lys
1               5


<210>  221
<211>  6
<212>  PRT
<213>  Artificial Sequence

<220>
<223>  Designed peptide

<400>  221

Asn Arg Val Lys Pro Arg
1               5


<210>  222
<211>  6
<212>  PRT
<213>  Artificial Sequence

<220>
<223>  Designed peptide

<400>  222

Asn Ser Ile Ala His Arg
1               5


<210>  223
<211>  6
<212>  PRT
<213>  Artificial Sequence

<220>
<223>  Designed peptide

<400>  223

Asn Ser Arg Tyr Phe Thr
1               5


<210>  224
<211>  6
<212>  PRT
<213>  Artificial Sequence

<220>
<223>  Designed peptide

<400>  224

Pro Asp Arg Phe Cys Ala
1               5

<210>  225
<211>  6
<212>  PRT
<213>  Artificial Sequence

<220>
<223>  Designed peptide

<400>  225

Pro Trp Thr Val Pro Asn
1               5


<210>  226
<211>  6
<212>  PRT
<213>  Artificial Sequence

<220>
<223>  Designed peptide

<400>  226

Gln Cys Glu Leu Lys Asp
1               5


<210>  227
<211>  6
<212>  PRT
<213>  Artificial Sequence

<220>
<223>  Designed peptide

<400>  227

Gln Phe Ser Gln Leu Gly
1               5


<210>  228
<211>  6
<212>  PRT
<213>  Artificial Sequence

<220>
<223>  Designed peptide

<400>  228

Gln Arg Val Ser Phe Leu
1               5


<210>  229
<211>  6
<212>  PRT
<213>  Artificial Sequence

<220>

<223>  Designed peptide

<400>  229

Gln Val Ile Gln Arg Leu
1               5


<210>  230
<211>  6
<212>  PRT
<213>  Artificial Sequence

<220>
<223>  Designed peptide

<400>  230

Gln Tyr Cys Phe Val Gly
1               5


<210>  231
<211>  6
<212>  PRT
<213>  Artificial Sequence

<220>
<223>  Designed peptide

<400>  231

Arg Gly Gly Arg Thr Leu
1               5


<210>  232
<211>  6
<212>  PRT
<213>  Artificial Sequence

<220>
<223>  Designed peptide

<400>  232

Arg Gly His Pro Ser Thr
1               5


<210>  233
<211>  6
<212>  PRT
<213>  Artificial Sequence

<220>
<223>  Designed peptide

<400>  233

Arg Gly Thr Met Trp Leu
1               5

<210>  234
<211>  6
<212>  PRT
<213>  Artificial Sequence

<220>
<223>  Designed peptide

<400>  234

Arg His Gly Gln Phe Phe
1               5


<210>  235
<211>  6
<212>  PRT
<213>  Artificial Sequence

<220>
<223>  Designed peptide

<400>  235

Arg His Thr Gln Thr Leu
1               5


<210>  236
<211>  6
<212>  PRT
<213>  Artificial Sequence

<220>
<223>  Designed peptide

<400>  236

Arg Ile Val Leu Glu Ser
1               5


<210>  237
<211>  6
<212>  PRT
<213>  Artificial Sequence

<220>
<223>  Designed peptide

<400>  237

Arg Lys Cys Ile Asp Arg
1               5


<210>  238
<211>  6
<212>  PRT
<213>  Artificial Sequence

<220>

<223>  Designed peptide

<400>  238

Arg Leu His Gln Cys Arg
1               5


<210>  239
<211>  6
<212>  PRT
<213>  Artificial Sequence

<220>
<223>  Designed peptide

<400>  239

Arg Leu Arg Asp Pro Gln
1               5


<210>  240
<211>  6
<212>  PRT
<213>  Artificial Sequence

<220>
<223>  Designed peptide

<400>  240

Arg Asn His Ser Arg Ile
1               5


<210>  241
<211>  6
<212>  PRT
<213>  Artificial Sequence

<220>
<223>  Designed peptide

<400>  241

Arg Ser Ser Ala Lys Arg
1               5


<210>  242
<211>  6
<212>  PRT
<213>  Artificial Sequence

<220>
<223>  Designed peptide

<400>  242

Arg Thr Ile Ser Gly Ser
1               5

<210>  243
<211>  6
<212>  PRT
<213>  Artificial Sequence

<220>
<223>  Designed peptide

<400>  243

Arg Thr Leu Leu Val Gln
1               5


<210>  244
<211>  6
<212>  PRT
<213>  Artificial Sequence

<220>
<223>  Designed peptide

<400>  244

Arg Thr Val Pro Ser Ser
1               5


<210>  245
<211>  6
<212>  PRT
<213>  Artificial Sequence

<220>
<223>  Designed peptide

<400>  245

Arg Tyr Met Pro Val Ala
1               5


<210>  246
<211>  6
<212>  PRT
<213>  Artificial Sequence

<220>
<223>  Designed peptide

<400>  246

Ser Ala Met Phe Ala Ser
1               5


<210>  247
<211>  6
<212>  PRT
<213>  Artificial Sequence

<220>

<223>  Designed peptide

<400>  247

Ser Glu His Asn Arg Tyr
1               5


<210>  248
<211>  6
<212>  PRT
<213>  Artificial Sequence

<220>
<223>  Designed peptide

<400>  248

Ser His Trp Arg Ser Tyr
1               5


<210>  249
<211>  6
<212>  PRT
<213>  Artificial Sequence

<220>
<223>  Designed peptide

<400>  249

Ser Ile Ile Thr Arg Arg
1               5


<210>  250
<211>  6
<212>  PRT
<213>  Artificial Sequence

<220>
<223>  Designed peptide

<400>  250

Ser Lys Val Ile Arg Trp
1               5


<210>  251
<211>  6
<212>  PRT
<213>  Artificial Sequence

<220>
<223>  Designed peptide

<400>  251

Ser Lys Tyr Thr Ala Leu
1               5

<210>  252
<211>  6
<212>  PRT
<213>  Artificial Sequence

<220>
<223>  Designed peptide

<400>  252

Ser Arg Ser His Trp Gly
1               5


<210>  253
<211>  6
<212>  PRT
<213>  Artificial Sequence

<220>
<223>  Designed peptide

<400>  253

Ser Ser Lys Ser Phe Ala
1               5


<210>  254
<211>  6
<212>  PRT
<213>  Artificial Sequence

<220>
<223>  Designed peptide

<400>  254

Ser Thr Glu Phe Ala Arg
1               5


<210>  255
<211>  6
<212>  PRT
<213>  Artificial Sequence

<220>
<223>  Designed peptide

<400>  255

Ser Thr Leu Val Cys Pro
1               5


<210>  256
<211>  6
<212>  PRT
<213>  Artificial Sequence

<220>

<223>  Designed peptide

<400>  256

Ser Thr Ser Val Asp Trp
1               5


<210>  257
<211>  6
<212>  PRT
<213>  Artificial Sequence

<220>
<223>  Designed peptide

<400>  257

Thr Asn Arg Thr Val Ser
1               5


<210>  258
<211>  6
<212>  PRT
<213>  Artificial Sequence

<220>
<223>  Designed peptide

<400>  258

Thr Asn Tyr Leu Ser Phe
1               5


<210>  259
<211>  6
<212>  PRT
<213>  Artificial Sequence

<220>
<223>  Designed peptide

<400>  259

Val Gly Met His His Ser
1               5


<210>  260
<211>  6
<212>  PRT
<213>  Artificial Sequence

<220>
<223>  Designed peptide

<400>  260

Val Met Ser Pro Cys Asn
1               5

<210>  261
<211>  6
<212>  PRT
<213>  Artificial Sequence

<220>
<223>  Designed peptide

<400>  261

Val Pro Phe Pro His His
1               5


<210>  262
<211>  6
<212>  PRT
<213>  Artificial Sequence

<220>
<223>  Designed peptide

<400>  262

Val Pro Leu Leu Ser Arg
1               5


<210>  263
<211>  6
<212>  PRT
<213>  Artificial Sequence

<220>
<223>  Designed peptide

<400>  263

Val Ser Asp Pro Val Val
1               5


<210>  264
<211>  6
<212>  PRT
<213>  Artificial Sequence

<220>
<223>  Designed peptide

<400>  264

Trp Thr Met Ser Pro Ala
1               5


<210>  265
<211>  6
<212>  PRT
<213>  Artificial Sequence

<220>

<223>  Designed peptide

<400>  265

Trp Val Phe Val Lys His
1               5


<210>  266
<211>  6
<212>  PRT
<213>  Artificial Sequence

<220>
<223>  Designed peptide

<400>  266

Tyr Gly Lys Gln Arg Gly
1               5


<210>  267
<211>  6
<212>  PRT
<213>  Artificial Sequence

<220>
<223>  Designed peptide

<400>  267

Tyr Lys Ala Ser His Gly
1               5


<210>  268
<211>  6
<212>  PRT
<213>  Artificial Sequence

<220>
<223>  Designed peptide

<400>  268

Tyr Arg Ser Asn His Gly
1               5


<210>  269
<211>  6
<212>  PRT
<213>  Artificial Sequence

<220>
<223>  Designed peptide

<400>  269

Tyr Tyr Pro Asp His Pro
1               5

<210>  270
<211>  50
<212>  DNA
<213>  Artificial Sequence

<220>
<223>  Synthetic oligonucleotide

<400>  270
cagacgtgtg ctcttccgat ctgatatcag atcggaagag cgtcgttaag                50


<210>  271
<211>  33
<212>  DNA
<213>  Artificial Sequence

<220>
<223>  Synthetic oligonucleotide

<400>  271
catcacaagt ttgtacaaaa aagcaggctg tga                                  33


<210>  272
<211>  30
<212>  DNA
<213>  Artificial Sequence

<220>
<223>  Synthetic oligonucleotide

<400>  272
tatcaccact ttgtacaaga aagctgggtt                                      30


<210>  273
<211>  31
<212>  DNA
<213>  Artificial Sequence

<220>
<223>  Synthetic oligonucleotide

<400>  273
atcacaagtt tgtactggga gggcgatcgc a                                    31


<210>  274
<211>  30
<212>  DNA
<213>  Artificial Sequence

<220>
<223>  Synthetic oligonucleotide

<400>  274
ctatcaccac tttgttcgct acctcgcgaa                                      30


<210>  275
<211>  28

<212>  DNA
<213>  Artificial Sequence

<220>
<223>  Synthetic oligonucleotide

<400>  275
acaagtggtg atagcttgtc gagaagta                                          28


<210>  276
<211>  29
<212>  DNA
<213>  Artificial Sequence

<220>
<223>  Synthetic oligonucleotide

<400>  276
gtacaaactt gtgatgatcc gcgcccgat                                         29


<210>  277
<211>  103
<212>  DNA
<213>  Artificial Sequence

<220>
<223>  Synthetic oligonucleotide

<400>  277
ccccaaataa tgattttatt ttgactgata gtgacctgtt cgttgcaaca aattgatgag      60

caatgctttt ttataatgcc aactttgtac aaaaaagcag gct                        103


<210>  278
<211>  105
<212>  DNA
<213>  Artificial Sequence

<220>
<223>  Synthetic oligonucleotide

<400>  278
agcttaccca gctttcttgt acaaagttgg cattataaga aagcattgct tatcaatttg      60

ttgcaacgaa caggtcacta tcagtcaaaa taaaatcatt atttg                      105


<210>  279
<211>  217
<212>  DNA
<213>  Artificial Sequence

<220>
<223>  Synthetic oligonucleotide


<220>
<221>  misc_feature
<222>  (136)..(137)
<223>  n is a, c, g, or t

<220>
<221>  misc_feature
<222>  (138)..(138)
<223>  n is g, or t

<220>
<221>  misc_feature
<222>  (139)..(140)
<223>  n is a, c, g, or t

<220>
<221>  misc_feature
<222>  (141)..(141)
<223>  n is g, or t

<220>
<221>  misc_feature
<222>  (142)..(143)
<223>  n is a, c, g, or t

<220>
<221>  misc_feature
<222>  (144)..(144)
<223>  n is g, or t

<220>
<221>  misc_feature
<222>  (145)..(146)
<223>  n is a, c, g, or t

<220>
<221>  misc_feature
<222>  (147)..(147)
<223>  n is g, or t

<220>
<221>  misc_feature
<222>  (148)..(149)
<223>  n is a, c, g, or t

<220>
<221>  misc_feature
<222>  (150)..(150)
<223>  n is g, or t

<220>
<221>  misc_feature
<222>  (151)..(152)
<223>  n is a, c, g, or t

<220>
<221>  misc_feature
<222>  (153)..(153)
<223>  n is g, or t

<400>  279
gatcccgcga aattaatacg actcactata ggggaagtat ttttacaaca attaccaaca      60

acaacaacaa acaacaacaa cattacattt tacattctac aactacaagc caccatgggc     120

agcggcggca gctgcnnnnn nnnnnnnnnn nnntgcgggg gaggcagcca tcatcatcat     180

catcacggcg gaagcaggac ggggggcggc gtggaaa                                217

<210>  280
<211>  117
<212>  DNA
<213>  Artificial Sequence

<220>
<223>  Synthetic oligonucleotide

<400>  280
gatcccgcga aattaatacg actcactata ggggaagtat ttttacaaca attaccaaca      60

acaacaacaa acaacaacaa cattacattt tacattctac aactacaagc caccatg        117

<210>  281
<211>  78
<212>  DNA
<213>  Artificial Sequence

<220>
<223>  Synthetic oligonucleotide

<220>
<221>  misc_feature
<222>  (38)..(39)
<223>  n is a, c, g, or t

<220>
<221>  misc_feature
<222>  (40)..(40)
<223>  n is g, or t

<220>
<221>  misc_feature
<222>  (41)..(42)
<223>  n is a, c, g, or t

<220>
<221>  misc_feature
<222>  (43)..(43)
<223>  n is g, or t

<220>
<221>  misc_feature
<222>  (44)..(45)
<223>  n is a, c, g, or t

<220>
<221>  misc_feature
<222>  (46)..(46)
<223>  n is g, or t

<220>
<221>  misc_feature
<222>  (47)..(48)
<223>  n is a, c, g, or t

<220>
<221>  misc_feature

<222>  (49)..(49)
<223>  n is g, or t

<220>
<221>  misc_feature
<222>  (50)..(51)
<223>  n is a, c, g, or t

<220>
<221>  misc_feature
<222>  (52)..(52)
<223>  n is g, or t

<220>
<221>  misc_feature
<222>  (53)..(54)
<223>  n is a, c, g, or t

<220>
<221>  misc_feature
<222>  (55)..(55)
<223>  n is g, or t

<400>  281
acaactacaa gccaccatgg gcagcggcgg cagctgcnnn nnnnnnnnnn nnnnntgcgg      60

gggaggcagc catcatca                                                    78


<210>  282
<211>  61
<212>  DNA
<213>  Artificial Sequence

<220>
<223>  Synthetic oligonucleotide

<400>  282
tttccacgcc gccccccgtc ctgcttccgc cgtgatgatg atgatgatgg ctgcctcccc      60

c                                                                      61


<210>  283
<211>  26
<212>  DNA
<213>  Artificial Sequence

<220>
<223>  Synthetic oligonucleotide


<220>
<221>  misc_feature
<222>  (1)..(1)
<223>  Biotin-TEG-dA

<220>
<221>  misc_feature
<222>  (3)..(3)
<223>  riboguanine

<220>

<221>  misc_feature
<222>  (12)..(12)
<223>  3-Cyanovinylcarbazole-dG

<220>
<221>  misc_feature
<222>  (23)..(23)
<223>  Amino C6-dT

<400>  283
nanaatttcc anccgccccc cgncct                                          26


<210>  284
<211>  60
<212>  DNA
<213>  Artificial Sequence

<220>
<223>  Synthetic oligonucleotide

<400>  284
gatcccgcga aattaatacg actcactata ggggaagtat ttttacaaca attaccaaca      60


<210>  285
<211>  22
<212>  DNA
<213>  Artificial Sequence

<220>
<223>  Synthetic oligonucleotide

<400>  285
tttccacgcc gccccccgtc ct                                              22


<210>  286
<211>  58
<212>  DNA
<213>  Artificial Sequence

<220>
<223>  Synthetic oligonucleotide

<400>  286
tcgtcggcag cgtcagatgt gtataagaga cagcattcta caactacaag ccaccatg        58


<210>  287
<211>  61
<212>  DNA
<213>  Artificial Sequence

<220>
<223>  Synthetic oligonucleotide

<400>  287
gtctcgtggg ctcggagatg tgtataagag acagtttcca cgccgccccc cgtcctgctt      60

c                                                                     61

<210>  288
<211>  47
<212>  DNA
<213>  Artificial Sequence

<220>
<223>  Synthetic oligonucleotide

<400>  288
caagcagaag acggcatacg agatactgag cggtctcgtg ggctcgg                    47


<210>  289
<211>  47
<212>  DNA
<213>  Artificial Sequence

<220>
<223>  Synthetic oligonucleotide

<400>  289
caagcagaag acggcatacg agattgcagc tagtctcgtg ggctcgg                    47


<210>  290
<211>  51
<212>  DNA
<213>  Artificial Sequence

<220>
<223>  Synthetic oligonucleotide

<400>  290
aatgatacgg cgaccaccga gatctacact ctctccgtcg tcggcagcgt c               51


<210>  291
<211>  51
<212>  DNA
<213>  Artificial Sequence

<220>
<223>  Synthetic oligonucleotide

<400>  291
aatgatacgg cgaccaccga gatctacacc gtctaattcg tcggcagcgt c               51


<210>  292
<211>  40
<212>  DNA
<213>  Artificial Sequence

<220>
<223>  Synthetic oligonucleotide


<220>
<221>  misc_feature
<222>  (1)..(1)
<223>  Biotin-TEG-dA

<220>

<221>  misc_feature
<222>  (21)..(21)
<223>  deoxyinosine

<220>
<221>  misc_feature
<222>  (27)..(27)
<223>  3-Cyanovinylcarbazole-dG

<220>
<221>  misc_feature
<222>  (38)..(38)
<223>  Amino C6-dT

<400>  292
naaaaaaaaa aaaaaaaaaa nttccanccg ccccccgnct                                40